# EUROPEAN PATENT APPLICATION

(11) **EP 4 714 480 A2**
(43) Date of publication of application: **25.03.2026**
(21) Application number: 26155279.8
(22) Date of filing: 01.09.2021
(51) Int. Cl.: A61M 5/32

(54) **DEVICES AND PROCESSES FOR DELIVERY OF THERAPEUTIC FLUIDS**

(30) Priority: 11.09.2020 US 202063077006 P
(62) Divisional of application: 21778300.0
(71) Applicant: Eli Lilly and Company, Indianapolis, IN 46285 (US)
(72) Inventor: ATTERBURY, William Godwin, Indianapolis, 46206-6288 (US); BLUM, Timothy Mark, Indianapolis, 46206-6288 (US); DAVIS, Yelena N., Indianapolis, 46206-6288 (US); HOLLEY, David Arthur, Indianapolis, 46206-6288 (US); TALLARICO, John Paul, Indianapolis, 46206-6288 (US); YOUNG, Jessica Diane, Indianapolis, 46206-6288 (US)
(74) Representative: J A Kemp LLP

(57) **Abstract**

A therapeutic agent delivery system includes a therapeutic agent delivery assembly carried by a housing. The therapeutic agent delivery assembly includes a chamber including a first passageway, a therapeutic agent carried in the first passageway, and a needle in communication with the first passageway. The therapeutic agent delivery assembly is translatable relative to the housing from a stowed configuration to a deployed configuration. In the deployed configuration the needle at least partially extends distally from the distal end portion of the housing. The therapeutic agent delivery assembly is translatable relative to the housing from the deployed configuration to a retracted configuration. In the retracted configuration the needle is disposed proximally relative to the distal end portion of the housing. A drive mechanism translates the therapeutic agent delivery assembly from the stowed configuration to the deployed configuration and from the deployed configuration to the retracted configuration.

## Description

### FIELD OF THE DISCLOSURE

The present disclosure relates to processes and devices for parenteral delivery of therapeutic agents. More particularly, the present disclosure relates to processes and devices for parenteral delivery of high-viscosity therapeutic fluids (for example, protein therapeutics).

### BACKGROUND OF THE DISCLOSURE

Protein therapeutics is an emerging class of drug therapy that provides treatment for a broad range of diseases, such as autoimmune disorders, cardiovascular diseases, diabetes, and cancer. A common delivery method for some protein therapeutics, such as monoclonal antibodies, is through intravenous infusion, in which large volumes of dilute solutions are delivered over time. Intravenous infusion usually requires the supervision of a doctor or nurse and is performed in a clinical setting. This can be inconvenient for a patient, and so efforts are being made to permit the delivery of protein therapeutics at home. Desirably, a protein therapeutic formulation can be administered using a syringe for subcutaneous delivery instead of requiring intravenous administration. Subcutaneous injections are commonly administered by laypersons, for example in the administration of insulin by diabetics.

Transitioning therapeutic protein formulations from intravenous delivery to injection devices like syringes and injection pens requires addressing challenges associated with delivering high concentrations of high molecular weight molecules in a manner that is easy, reliable, and causes minimal pain to the patient. In this regard, while intravenous bags typically have a volume of 1 liter, the standard volume for a syringe ranges from 0.3 milliliters up to 25 milliliters. Thus, depending on the drug, to deliver the same amount of therapeutic proteins, the concentration may have to increase by a factor of 40 or more. Also, injection therapy is moving towards smaller needle diameters and faster delivery times for purposes of patient comfort and compliance.

Delivery of protein therapeutics is also challenging because of the high viscosity associated with such therapeutic formulations, and the high forces needed to push such formulations through a parenteral device. Formulations with absolute viscosities above 40-60 centipoise (cP) may be difficult to deliver by conventional spring driven auto-injectors for multiple reasons. Structurally, the footprint of a spring for the amount of pressure delivered is relatively large and fixed to specific shapes, which reduces flexibility of design for delivery devices. Next, auto-injectors are usually made of plastic parts. However, a large amount of energy must be stored in the spring to reliably deliver high-viscosity fluids. If not properly designed, this stored energy may cause damage to the plastic parts due to creep, which is the tendency of the plastic part to permanently deform under stress. An auto-injector typically operates by using the spring to push a needle-containing internal component towards an outer edge of the housing of the syringe. The sound associated with the operation of a spring-based auto-injector may cause patient anxiety, potentially reducing future compliance. The generated pressure versus time profile of such a spring driven auto-injector cannot be readily modified, which prevents users from fine tuning pressure to meet their delivery needs.

It would be desirable to provide processes and devices by which a therapeutic fluid, in particular a high-viscosity fluid, could be self-administered in a reasonable time and with a limited injection space. These processes and devices could be used to deliver high-concentration protein, high-viscosity pharmaceutical formulations, or other therapeutic fluids.

### SUMMARY

According to an embodiment of the present disclosure, a therapeutic agent delivery system includes a housing having a distal end portion. A therapeutic agent delivery assembly is carried by the housing, and the therapeutic agent delivery assembly includes a chamber including a first passageway, a therapeutic agent carried in the first passageway, and a needle in communication with the first passageway. The therapeutic agent delivery assembly is translatable relative to the housing from a stowed configuration to a deployed configuration. In the deployed configuration the needle at least partially extends distally from the distal end portion of the housing. The therapeutic agent delivery assembly is translatable relative to the housing from the deployed configuration to a retracted configuration. In the retracted configuration the needle is disposed proximally relative to the distal end portion of the housing. A drive mechanism is carried by the housing, and the drive mechanism includes a torsion spring, a first release device, and a second release device. The first release device is actuatable to permit the torsion spring to release energy and reconfigure from a higher energy storage configuration to an intermediate energy storage configuration, and the drive mechanism thereby translates the therapeutic agent delivery assembly from the stowed configuration to the deployed configuration. The second release device is actuatable to permit the torsion spring to further release energy and reconfigure from the intermediate energy storage configuration to a lower energy storage configuration, and the drive mechanism thereby translates the therapeutic agent delivery assembly from the deployed configuration to the retracted configuration.

According to another embodiment of the present disclosure, a therapeutic agent delivery system includes a housing having a distal end portion. A therapeutic agent delivery assembly is carried by the housing, and the therapeutic agent delivery assembly includes a chamber including a first passageway, a therapeutic agent carried in the first passageway, and a needle in communication with the first passageway. The therapeutic agent delivery assembly is translatable relative to the housing from a stowed configuration to a deployed configuration. In the deployed configuration the needle at least partially extends distally from the distal end portion of the housing. The therapeutic agent delivery assembly is translatable relative to the housing from the deployed configuration to a retracted configuration. In the retracted configuration the needle is disposed proximally relative to the distal end portion of the housing. A drive mechanism is carried by the housing. The drive mechanism includes a threaded nut and a drive screw carried by and threadably coupled to the threaded nut. The drive screw is rotatable and translatable relative to the housing to translate the therapeutic agent delivery assembly relative to the housing. The drive mechanism further includes a torsion spring. The torsion spring releases energy to rotate and translate the drive screw relative to the housing, and the drive mechanism thereby translates the therapeutic agent delivery assembly from the stowed configuration to the deployed configuration and from the deployed configuration to the retracted configuration.

According to yet another embodiment of the present disclosure, a therapeutic agent delivery system includes a housing having a distal end portion. A therapeutic agent delivery assembly is carried by the housing, and the therapeutic agent delivery assembly includes a chamber including a first passageway, a therapeutic agent carried in the first passageway, and a needle in communication with the first passageway. The therapeutic agent delivery assembly is translatable relative to the housing from a stowed configuration to a deployed configuration. In the deployed configuration the needle at least partially extends distally from the distal end portion of the housing. The therapeutic agent delivery assembly is translatable relative to the housing from the deployed configuration to a retracted configuration. In the retracted configuration the needle is disposed proximally relative to the distal end portion of the housing. A drive mechanism is carried by the housing. The drive mechanism includes a retraction cap, a threaded nut carried by and threadably coupled to the retraction cap, and a drive screw carried by and threadably coupled to the threaded nut. The drive screw is rotatable and translatable relative to the housing to translate the therapeutic agent delivery assembly relative to the housing. The drive mechanism further includes a rotary actuator configured to drive the drive screw in a first rotary direction. The drive screw includes a first external threaded surface, the threaded nut includes a first internal threaded surface that threadably engages the first external threaded surface, and both of the first external threaded surface and the first internal threaded surface have a first thread direction. The threaded nut further includes a second external threaded surface, the retraction cap includes a second internal threaded surface that threadably engages the second external threaded surface, and both of the second external threaded surface and the second internal threaded surface have a second thread direction opposite the first thread direction. The rotary actuator is configured to drive the drive screw in the first rotary direction and first thereby translate the therapeutic agent delivery assembly from the stowed configuration to the deployed configuration due to the first thread direction, and second thereby translate the therapeutic agent delivery assembly from the deployed configuration to the retracted configuration due to the second thread direction.

### BRIEF DESCRIPTION OF THE DRAWINGS

The above-mentioned and other features and advantages of this disclosure, and the manner of attaining them, will become more apparent and will be better understood by reference to the following description of embodiments of the invention taken in conjunction with the accompanying drawings, wherein:
FIG. 1 is a top perspective view of a therapeutic agent delivery system according to an embodiment of the present disclosure.
FIG. 2 is a partially exploded view of the therapeutic agent delivery system of FIG. 1.
FIG. 3 is a longitudinal sectional view of the therapeutic agent delivery system of FIG. 1.
FIG. 4 is a top perspective view of a proximal housing portion of a housing of the therapeutic agent delivery system of FIG. 1.
FIG. 5 is a bottom perspective view of the proximal housing portion of FIG. 4.
FIG. 6 is a longitudinal sectional view of the proximal housing portion along line 6-6 of FIG. 4.
FIG. 7 is a top perspective view of a distal housing portion of the housing of the therapeutic agent delivery system of FIG. 1.
FIG. 8 is a bottom perspective view of the distal housing portion of FIG. 7.
FIG. 9 is a top perspective view of a user input support of the therapeutic agent delivery system of FIG. 1.
FIG. 10 is a top perspective view of a user input of the therapeutic agent delivery system of FIG. 1.
FIG. 11 is a top perspective view of a first latch device and a wire of the therapeutic agent delivery system of FIG. 1.
FIG. 12 is a top perspective view of a first catch device of the therapeutic agent delivery system of FIG. 1.
FIG. 13 is a longitudinal sectional view of the first catch device along line 13-13 of FIG. 12.
FIG. 14 is a top perspective view of a rotary actuator of the therapeutic agent delivery system of FIG. 1.
FIG. 15 is a bottom perspective view of the rotary actuator of FIG. 14.
FIG. 16 is a top perspective view of a drive screw of the therapeutic agent delivery system of FIG. 1.
FIG. 17 is a bottom perspective view of the drive screw of FIG. 16.
FIG. 18 is a top perspective view of a drive screw coupler of the therapeutic agent delivery system of FIG. 1.
FIG. 19 is a longitudinal sectional view of the drive screw coupler along line 19-19 of FIG. 18.
FIG. 20 is a top perspective view of a pressure generating actuator of a therapeutic agent delivery assembly of the therapeutic agent delivery system of FIG. 1.
FIG. 21 is a bottom perspective view of the pressure generating actuator of FIG. 20.
FIG. 22 is a partially exploded perspective view of the pressure generating actuator of FIG. 20.
FIG. 23 is a longitudinal sectional view of the pressure generating actuator along line 23-23 of FIG. 20.
FIG. 24 is a side view of a syringe assembly of the therapeutic agent delivery assembly of the therapeutic agent delivery system of FIG. 1.
FIG. 25 is a longitudinal sectional view of the syringe assembly along line 25-25 of FIG. 24.
FIG. 26 is a top perspective view of a restraint device of the therapeutic agent delivery system of FIG. 1.
FIG. 27 is a bottom perspective view of the restraint device of FIG. 26.
FIG. 28 is a top perspective view of a second latch device of the therapeutic agent delivery system of FIG. 1.
FIG. 29 is a top perspective view of a second catch device of the therapeutic agent delivery system of FIG. 1.
FIG. 30 is a longitudinal sectional view of the second catch device along line 30-30 of FIG. 29.
FIG. 31 is a top perspective view of a retraction cap of the therapeutic agent delivery system of FIG. 1.
FIG. 32 is a longitudinal sectional view of the retraction cap along line 32-32 of FIG. 31.
FIG. 33 is a schematic representation of an electronics assembly of the therapeutic agent delivery system of FIG. 1.
FIG. 34 is a top perspective view of the electronics assembly of FIG. 33.
FIG. 35 is a longitudinal sectional view of the therapeutic agent delivery system of FIG. 1 in an initial or first configuration.
FIG. 36 is a detail top perspective view of a proximal end portion of the therapeutic agent delivery system of FIG. 1 in the first configuration; several external components are shown in hidden lines to illustrate internal components.
FIG. 37 is a detail top perspective view of the proximal end portion of the therapeutic agent delivery system of FIG. 1 upon actuation of a first release device; several external components are shown in hidden lines to illustrate internal components.
FIG. 38 is another detail top perspective view of the proximal end portion of the therapeutic agent delivery system of FIG. 1 upon actuation of the first release device; several external components are shown in hidden lines to illustrate internal components.
FIG. 39 is another detail top perspective view of the proximal end portion of the therapeutic agent delivery system of FIG. 1 upon a drive screw engaging a second catch device; several external components are shown in hidden lines to illustrate internal components.
FIG. 40 is a longitudinal sectional view of the therapeutic agent delivery system of FIG. 1 in a deployed configuration.
FIG. 41 is a longitudinal sectional view of a shuttle of the pressure generating actuator being rotated relative to first and second mixing chambers of the pressure generating actuator and thereby actuating the actuator.
FIG. 42 is a longitudinal sectional view of the shuttle and a mixing piston of the pressure generating actuator in a deployed configuration.
FIG. 43 is a longitudinal sectional view of the therapeutic agent delivery system of FIG. 1 upon a syringe piston moving in a syringe passageway to discharge a therapeutic agent from the needle.
FIG. 44 is a cross sectional view of the therapeutic agent delivery system along line 44-44 of FIG. 43 and through a second latch device and a second catch device in an initial configuration.
FIG. 45 is a detail top perspective view of the proximal end portion of the therapeutic agent delivery system of FIG. 1 upon actuation of a second release device; several external components are shown in hidden lines to illustrate internal components.
FIG. 46 is another detail top perspective view of the proximal end portion of the therapeutic agent delivery system of FIG. 1 upon actuation of the second release device; several external components are shown in hidden lines to illustrate internal components.
FIG. 47 is yet another detail top perspective view of the proximal end portion of the therapeutic agent delivery system of FIG. 1 upon actuation of the second release device; several external components are shown in hidden lines to illustrate internal components.
FIG. 48 is a longitudinal sectional view of the therapeutic agent delivery system of FIG. 1 in a retracted configuration.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

The present disclosure relates to systems, devices, and processes for parenteral delivery of therapeutic agents, such as high-viscosity therapeutic fluids. Such systems and devices are illustratively provided with relatively compact profiles.

### 1. Drugs/Therapeutic Agents

Systems and devices according to the present disclosure may carry and facilitate delivery of a drug to a subject. The term "drug" refers to one or more therapeutic agents including but not limited to insulins, insulin analogs such as insulin lispro or insulin glargine, insulin derivatives, GLP-1 receptor agonists such as dulaglutide or liraglutide, glucagon, glucagon analogs, glucagon derivatives, gastric inhibitory polypeptide (GIP), GIP analogs, GIP derivatives, combined GIP/GLP-1 agonists such as tirzepatide, oxyntomodulin analogs, oxyntomodulin derivatives, therapeutic antibodies and any therapeutic agent that is capable of delivery by devices according to the present disclosure. The drug may be formulated with one or more excipients. Devices according to the present disclosure are operated in a manner generally as described herein by a patient, caregiver or healthcare professional to deliver a drug to a subject.

In certain embodiments, a therapeutic agent is protein, such as a monoclonal antibody or some other protein which is therapeutically useful. In some embodiments, the protein may have a concentration of from about 75 mg/mL to about 500 mg/mL in a fluid. In certain embodiments, the protein may have a concentration of about 150 mg/mL, 200 mg/mL, 250 mg/mL, or more. A drug may further contain a solvent or non-solvent, such as water, perfluoroalkane solvent, safflower oil, or benzyl benzoate.

A drug may be a fluid, more specifically a high-viscosity fluid and may have an absolute viscosity of from about 5 cP to about 1000 cP. In certain embodiments, a high-viscosity fluid has an absolute viscosity of at least about 10 cP, 20 cP, 30 cP, 40 cP, 50 cP, 60 cP, or more.

### 2. Therapeutic Agent Delivery System

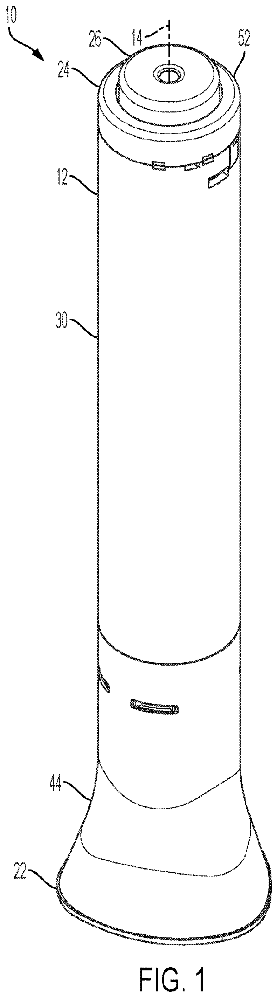

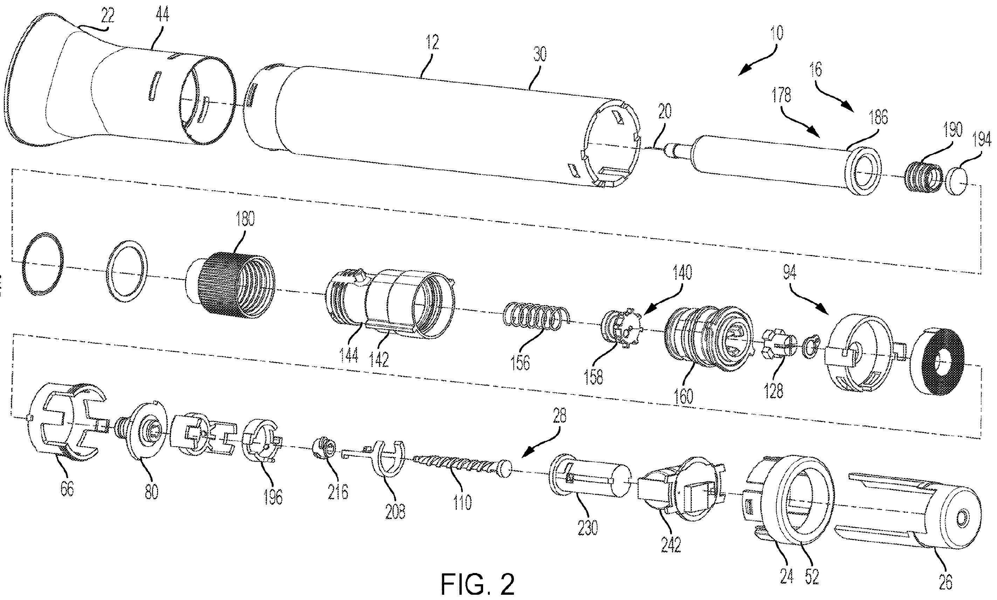

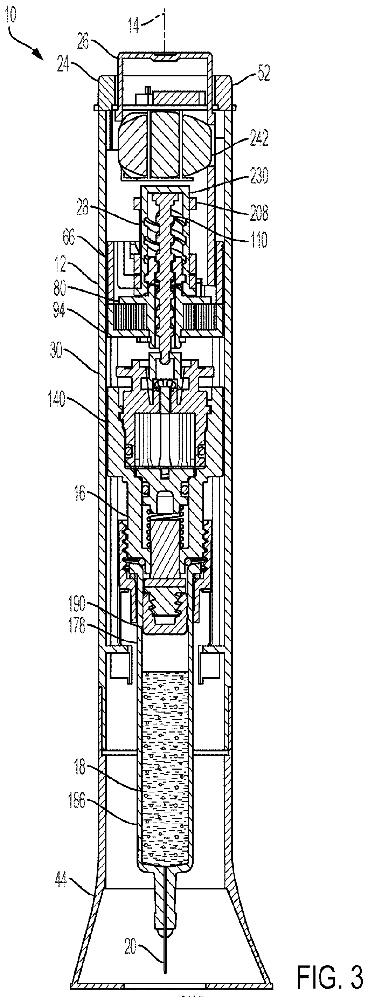

FIGS. 1-3 illustrate a therapeutic agent delivery system 10 according to an embodiment of the present disclosure. Illustratively, the therapeutic agent delivery system 10 generally includes the profile of an auto-injector pen, although other profiles may alternatively be used. Generally, the therapeutic agent delivery system 10 includes a housing 12 that is elongated along a longitudinal axis 14. The housing 12 carries a therapeutic agent delivery assembly 16. The therapeutic agent delivery assembly 16 includes a therapeutic agent 18 (shown in FIG. 3) and a needle 20, and the therapeutic agent delivery assembly 16 translates relative to the housing 12 from a stowed configuration (as illustratively shown in FIGS. 1-3, a configuration in which the needle 20 is disposed entirely within the housing 12) to a deployed configuration (shown elsewhere - for example, a configuration in which the needle 20 is at least partially exposed at a distal end portion 22 of the housing 12 and configured to engage the subject and deliver the therapeutic agent to the subject). A proximal end portion 24 of the therapeutic agent delivery system 10 includes a user input 26 (illustratively, a depressible button) that is actuated to actuate a drive mechanism 28, and the drive mechanism 28 in turn actuates the therapeutic agent delivery assembly 16 (that is, move the needle 20 from the stowed configuration to the deployed configuration and deliver the therapeutic agent to the user). After actuation, the drive mechanism 28 translates the therapeutic agent delivery assembly 16 relative to the housing 12 from the deployed configuration to a retracted configuration (shown elsewhere - for example, a configuration in which the needle 20 is disposed entirely within the therapeutic agent delivery system 10). After reaching the retracted configuration, the therapeutic agent delivery assembly 16 is inhibited from being translated to the deployed configuration (stated another way, the system 10 is "locked out"). These aspects, features, and components of the therapeutic agent delivery system 10 are described in further detail below.

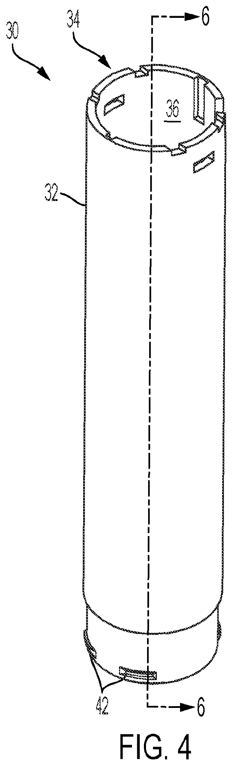

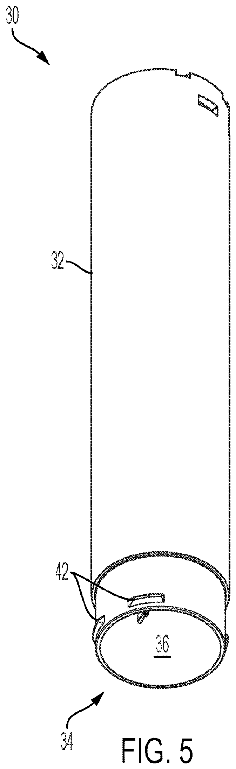

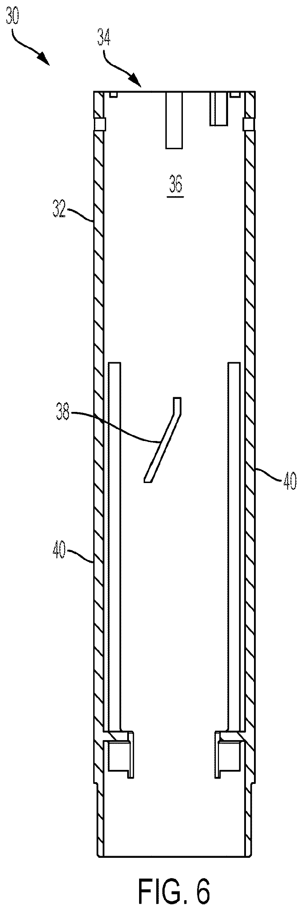

FIGS. 4-6 illustrate a proximal housing portion 30 of the housing 12. The proximal housing portion 30 includes a main body 32 that has a generally cylindrical shape. The main body 32 includes an inner passageway 34 that carries other components of the therapeutic agent delivery system 10. Adjacent to the inner passageway 34, an inner surface 36 of the proximal housing portion 30 carries an actuation feature (illustratively, two helically extending ramps 38, one of which is shown FIG. 6) that, as described in further detail below, selectively engage and facilitate actuating the therapeutic agent delivery assembly 16 (shown elsewhere). The inner surface 36 of the proximal housing portion 30 carries translation features (illustratively, two pairs of axially extending ridges 40, one ridge 40 of each pair being shown in FIG. 6) that facilitate translation of the therapeutic agent delivery assembly 16 relative to the proximal housing portion 30. The proximal housing portion 30 includes a coupling feature (illustratively, a plurality of snap connectors 42) for coupling to another portion of the housing 12.

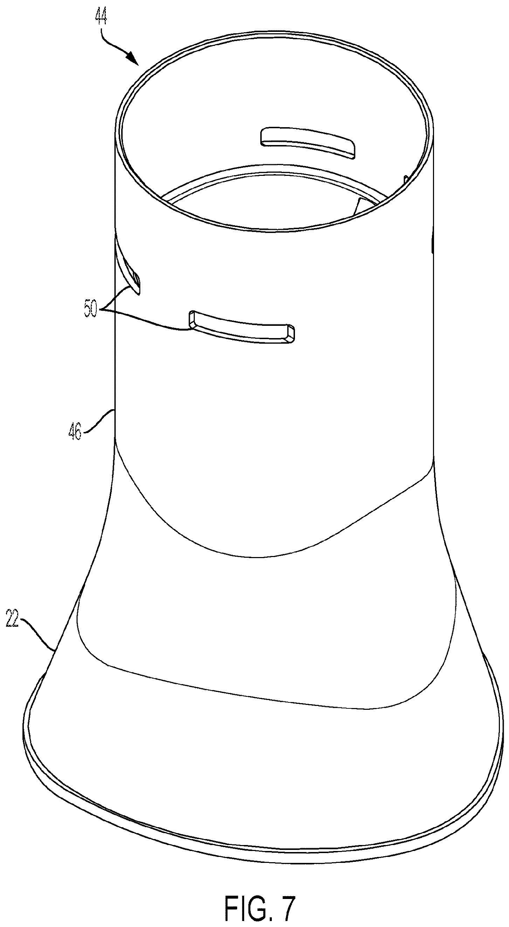

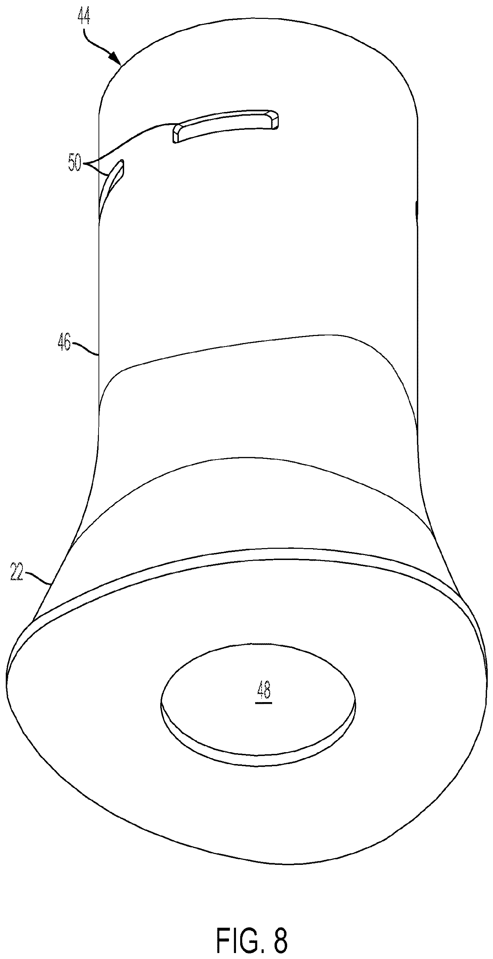

FIGS. 7 and 8 illustrate a distal housing portion 44 of the housing 12. The distal housing portion 44 includes a main body 46 that has a generally conical shape with a flared distal end portion 22. The main body 46 includes an inner passageway 48 that carries the therapeutic agent delivery assembly 16 (shown elsewhere). The distal housing portion 44 also includes a coupling feature (illustratively, a plurality of apertures 50) for coupling to the coupling feature of the proximal housing portion 30 (shown elsewhere - illustratively, the plurality of snap connectors 42). In other embodiments, different arrangements of the distal housing portion 44 are possible. For example, the distal housing portion 44 may be monolithically formed with the proximal housing portion 30.

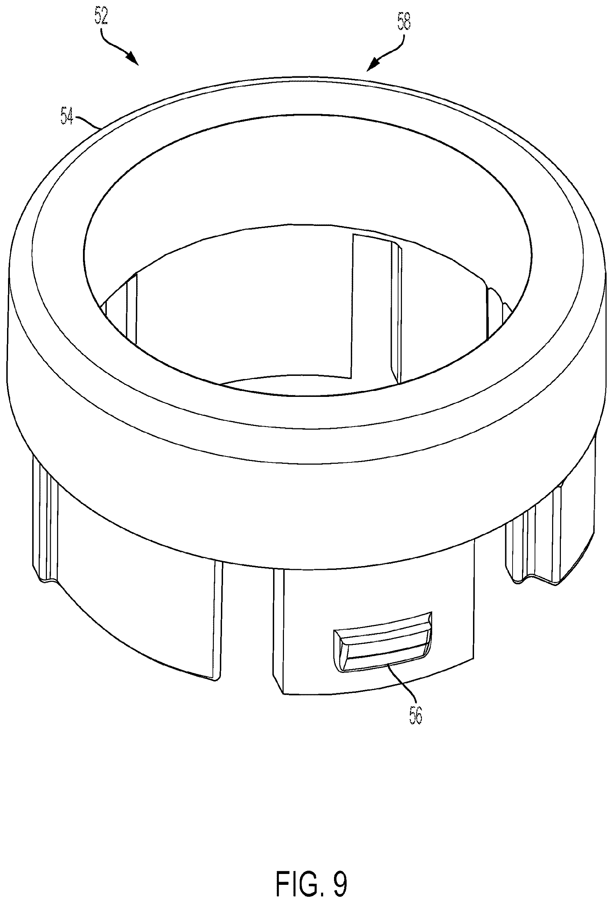

FIG. 9 illustrates a user input support 52 of the therapeutic agent delivery system 10. The user input support 52 couples to the proximal housing portion 30 opposite the distal housing portion 44 (both shown elsewhere). The user input support 52 includes a main body 54, and the main body 54 carries a coupling feature (illustratively, a plurality of snap connectors 56) for coupling to the proximal housing portion 30. The main body 54 includes an inner passageway 58 that receives the user input 26 (shown elsewhere). In other embodiments, different arrangements of the user input support 52 are possible.

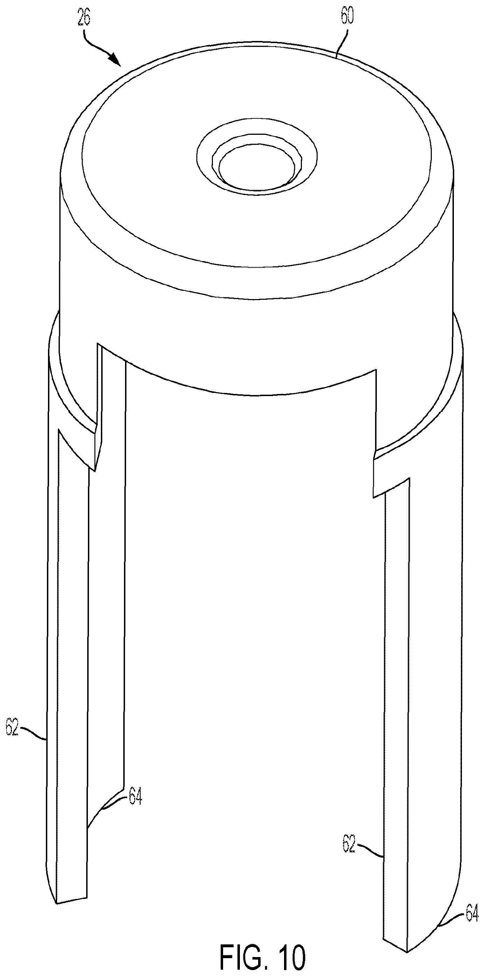

FIG. 10 illustrates the user input 26 of the therapeutic agent delivery system 10. The user input 26 is translatably carried by user input support 52 (shown elsewhere). The user input 26 includes an exposed portion 60 that is pressed by a user to translate the user input 26 relative to the user input support 52 and the housing 12 (shown elsewhere). The user input 26 also includes an actuation feature that facilitates actuating the drive mechanism 28 and the therapeutic agent delivery assembly 16 (both shown elsewhere). Illustratively, the actuation feature includes two arms 62 that are disposed opposite the exposed portion 60. Each of the arms 62 includes an actuation surface (illustratively, a distally facing surface 64). Interaction of the arms 62 with other components of the therapeutic agent delivery system 10 is described in further detail below. In other embodiments, different arrangements of the user input 26 are possible.

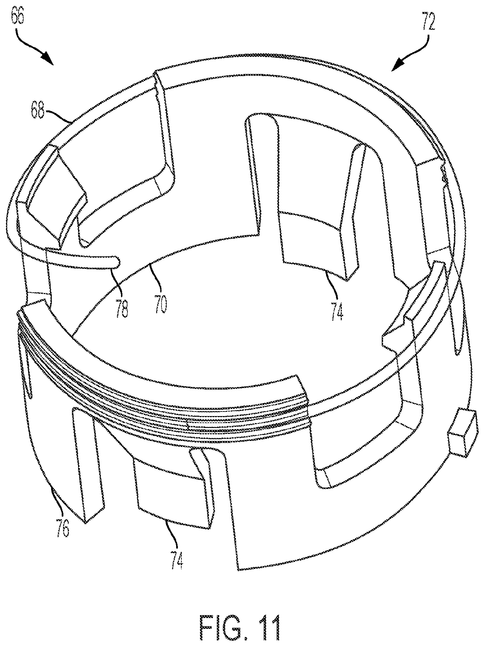

FIG. 11 illustrates a first latch device 66 and a wire 68 of the drive mechanism 28. The first latch device 66 includes a main body 70 that has a generally annular shape. The main body 70 includes an inner passageway 72 that carries other components of the drive mechanism 28. The main body 70 also includes a latch feature. Illustratively, the latch feature is provided by two diametrically opposed, distally and radially inwardly extending legs 74. As described in further detail below, the legs 74 deflect radially outwardly to facilitate actuating the drive mechanism 28. An outer surface 76 of the main body 70 carries the wire 68. Illustratively, the wire 68 extends about a portion of the outer surface 76 and forms a loop 78 in the inner passageway 72 of the first latch device 66. The wire 68 contracts or otherwise shortens to facilitate actuation of the drive mechanism 28. Further details of the wire 68 are described below. In other embodiments, different arrangements of the first latch device 66 and the wire 68 are possible.

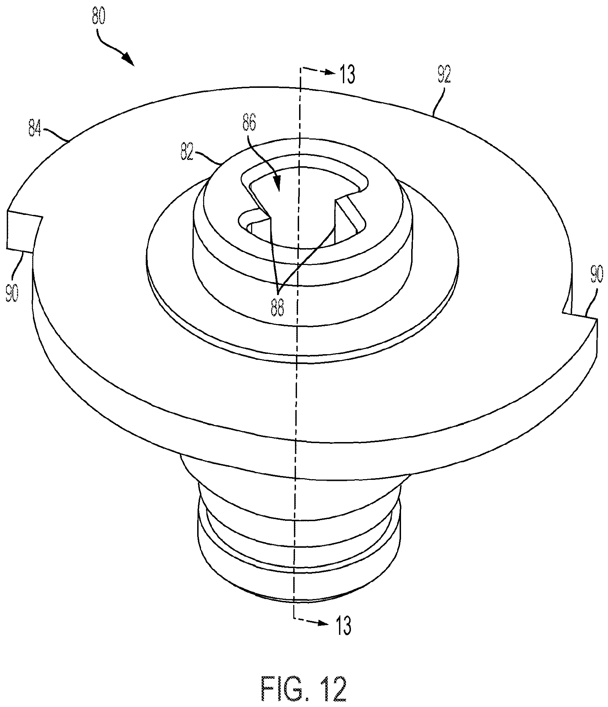

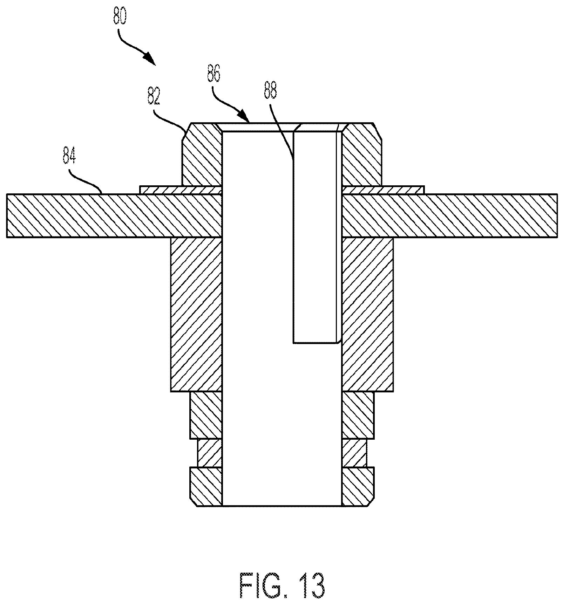

FIGS. 12 and 13 illustrate a first catch device 80 of the drive mechanism 28. The first catch device 80 includes a shaft 82 that carries an enlarged wheel 84. The shaft 82 includes an inner passageway 86, and the inner passageway 86 includes a translation feature (illustratively, two longitudinally extending shoulder surfaces 88) for slidably carrying and rotatably securing the first catch device 80 to another component of the drive mechanism 28 (shown elsewhere). The enlarged wheel 84 is disposed around the shaft 82. The enlarged wheel 84 includes a catch feature (illustratively, two transversely facing surfaces 90 disposed on the outer perimeter 92 of the wheel 84) for detachably engaging the latch feature of the first latch device 66 (shown elsewhere - illustratively, the radially inwardly extending legs 74). In other embodiments, different arrangements of the first catch device 80 are possible.

As described in further detail below, the first latch device 66 and the first catch device 80 together form a first release device of the drive mechanism 28. More specifically, the latch feature of the first latch device 66 (illustratively, the radially inwardly extending legs 74) may detach from or release the catch feature of the first catch device 80 (illustratively, the two transversely facing surfaces 90 of the wheel 84) to facilitate actuation of the drive mechanism 28, more specifically to facilitate reconfiguration of the system 10 from the stowed configuration to the deployed configuration.

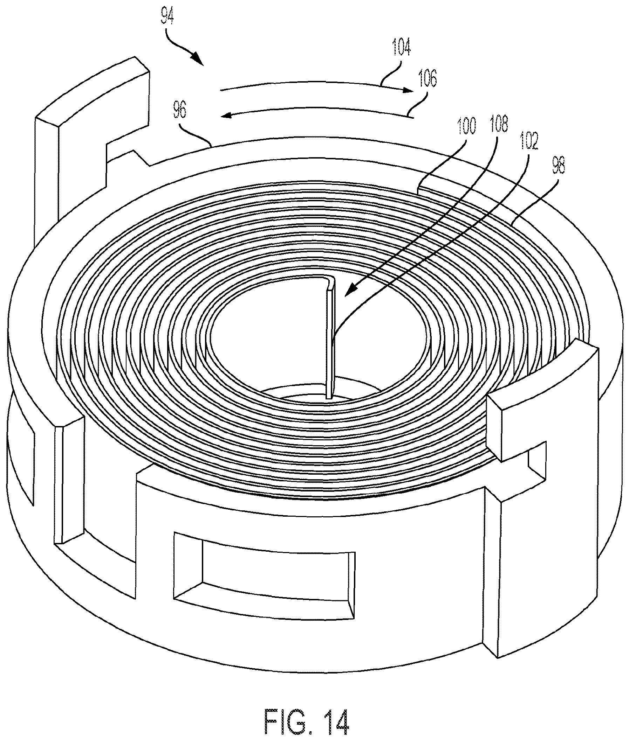

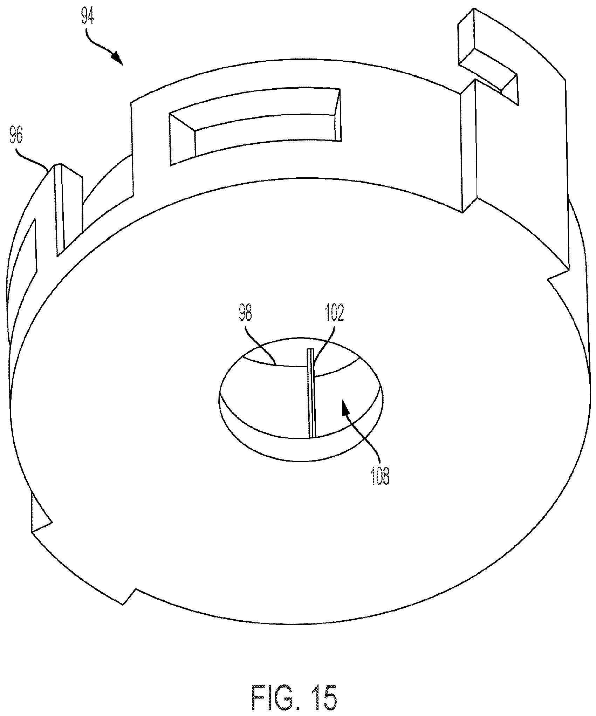

FIGS. 14 and 15 illustrate a rotary actuator 94 of the drive mechanism 28. The rotary actuator 94 illustratively includes a support frame 96 that carries a torsion spring 98. The torsion spring 98 may specifically be, for example, a spiral-wound torsion spring, which may also be referred to as a power spring or a clock spring. The torsion spring 98 includes a first end 100 (shown in FIG. 14) that is secured to the support frame 96 and a movable second end 102. The second end 102 is secured to the shaft 82 of the first catch device 80 (shown elsewhere). The second end 102 translates along a circular path relative to the support frame 96 as the torsion spring 98 winds and unwinds. More specifically, the second end 102 translates along the circular path in a first direction 104 as the torsion spring 98 winds upon itself to store energy, and the second end 102 translates along the circular path in an opposite section direction 106 as the torsion spring 98 unwinds and releases energy. The second end 102 of the torsion spring 98 is disposed adjacent to an inner passageway 108 that extends through both the torsion spring 98 and the support frame 96. As described in further detail below, the inner passageway 108 receives other components of the drive mechanism 28. In other embodiments, other arrangements of the rotary actuator 94 are possible.

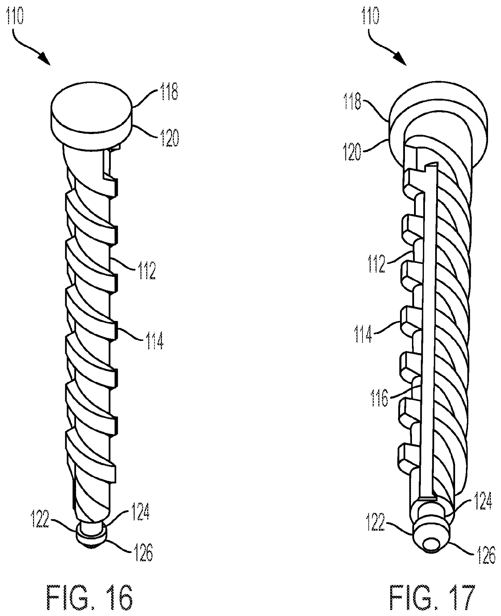

FIGS. 16 and 17 illustrate a drive screw 110 of the drive mechanism 28. The drive screw 110 includes an elongated shaft 112, and the shaft 112 includes a drive feature. The drive feature is illustratively provided as a first external threaded surface 114 for threadably engaging another component of the drive mechanism 28, as described further below. The first external threaded surface 114 has a first thread direction, illustratively a left-handed thread direction. The first external threaded surface 114 is interrupted by two longitudinally extending grooves or cutouts 116, one of which is shown in FIG. 17. The cutouts 116 facilitate slidably coupling and rotatably securing the drive screw to the translation feature of the first catch device 80 (shown elsewhere - illustratively, the longitudinally extending shoulder surfaces 88 within the inner passageway 86). At a proximal end portion 118, the drive screw 110 includes an enlarged head 120. As described in further detail below, the enlarged head 120 acts as a stop feature to inhibit movement of the drive screw 110 relative to other components of the drive mechanism 28. At a distal end portion 122, the drive screw 110 includes a coupling feature (illustratively, a narrow shank 124 carrying an enlarged tip 126) for rotatably coupling and translatably securing the drive screw 110 to another component of the drive mechanism 28, as described in further detail below. In other embodiments, other arrangements of the drive screw 110 are possible.

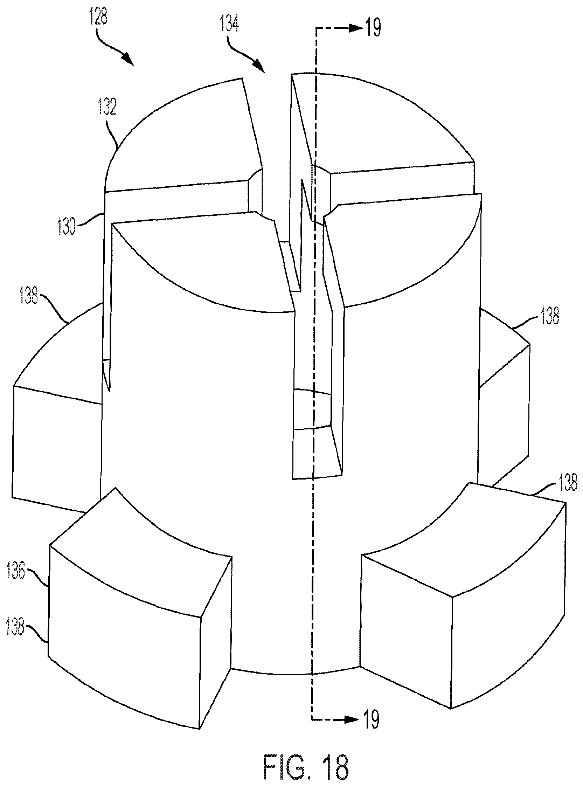

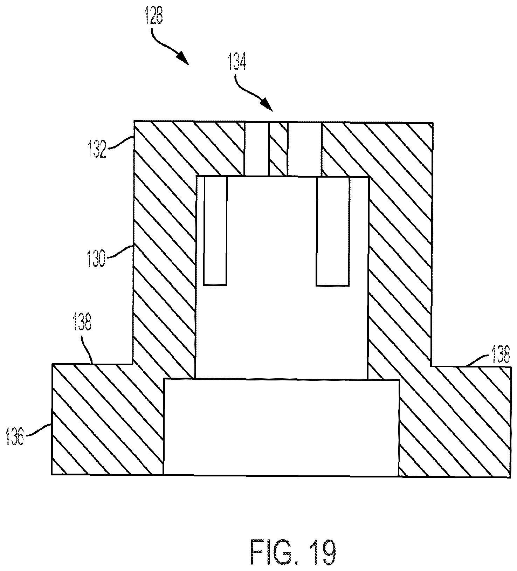

FIGS. 18 and 19 illustrate a drive screw coupler 128 of the drive mechanism 28. The drive screw coupler 128 includes a main body 130, and a distal end portion 132 of the main body 130 includes a coupling feature (illustratively, an opening 134) for rotatably coupling and translatably securing to the coupling feature of the drive screw 110 (shown elsewhere - illustratively, receiving the narrow shank 124 and the enlarged tip 126). A proximal end portion 136 of the main body 130 includes a coupling feature (illustratively, a plurality of ledges 138 or radially-outwardly extending L-shaped protrusions 138) that couples the drive screw coupler 128 to the therapeutic agent delivery assembly 16 (shown elsewhere). In other embodiments, different arrangements of the drive screw coupler 128 are possible.

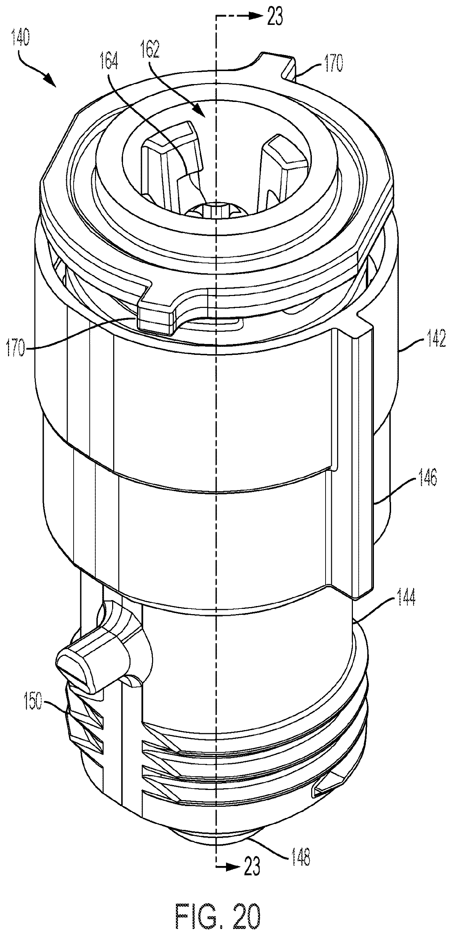

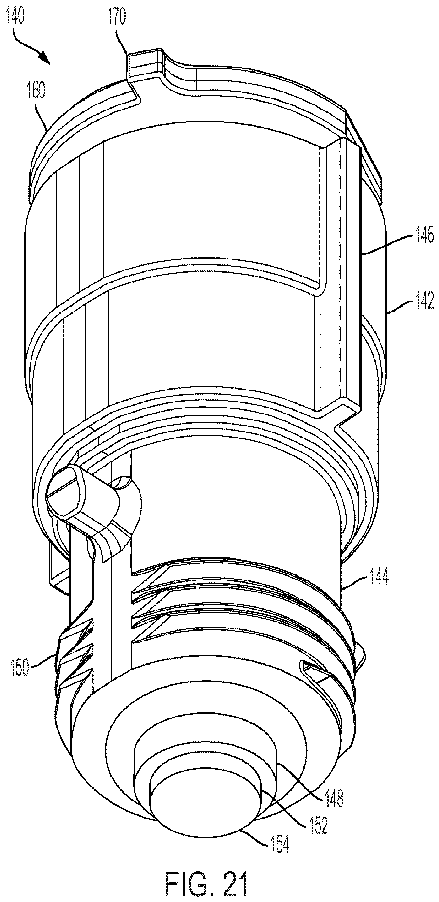

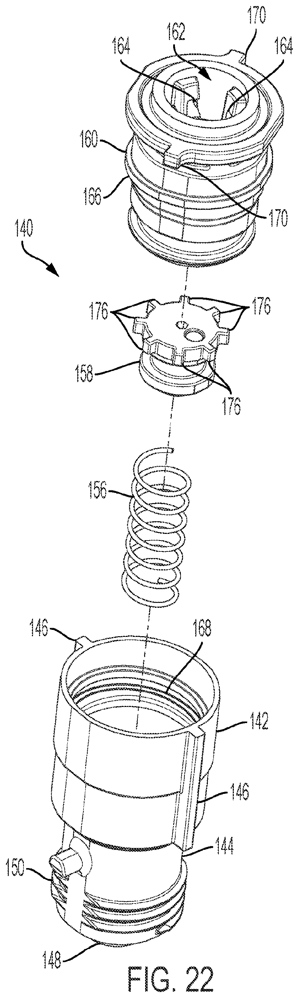

FIGS. 20-23 illustrate a pressure generating actuator 140 of the therapeutic agent delivery assembly 16. Generally, the pressure generating actuator 140 is actuated by the user input 26, via the drive mechanism 28, to facilitate mixing of internally-carried chemical reagents, which generates one or more pressurized fluids (for example, one or more gases). Examples of suitable reagents and generated gases are provided below. As described in further detail below, the pressurized fluid(s) are delivered to and facilitate movement of other components of the therapeutic agent delivery assembly 16.

The pressure generating actuator 140 includes a first mixing chamber 142 and a second mixing chamber 144, which are illustratively monolithically formed with each other. Externally, the first mixing chamber 142 and the second mixing chamber 144 include translation features (illustratively, two axially extending ridges 146) for translatably coupling to the translation features of the proximal housing portion 30 (shown elsewhere - illustratively, each of the axially extending ridges 146 is translatably received by one of the pairs of axially extending ridges 40 of the proximal housing portion 30). As a result, the pressure generating actuator 140 is translatably carried by the proximal housing portion 30. At an outlet end portion 148, the mixing chambers 142, 144 include an outlet coupling feature (illustratively, an externally threaded surface 150) for coupling to another component of the therapeutic agent delivery assembly 16. The outlet end portion 148 also includes an actuator outlet 152 (illustratively shown as carrying an absorbent material 154, as described in further detail below). Pressurized fluid is discharged from the pressure generating actuator 140 via the outlet 152.

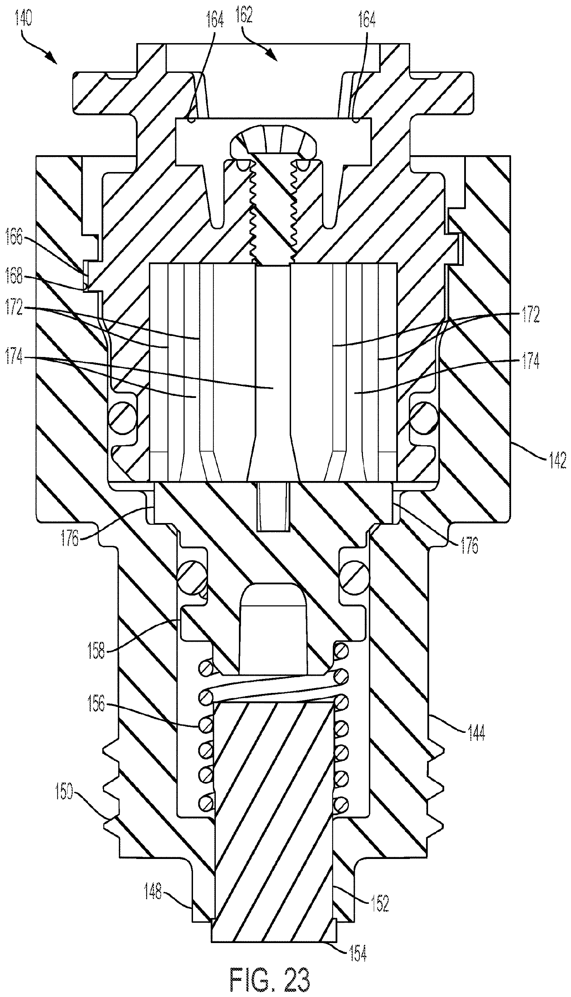

Internally, the mixing chambers 142, 144 carry an actuator spring 156, a mixing piston 158, and a rotatable shuttle 160 in an axially stacked arrangement. The rotatable shuttle 160 includes a recess 162, and the recess 162 carries a coupling feature (illustratively, a plurality of ledges 164 or radially-outwardly extending L-shaped protrusions 164) that engages the coupling feature of the drive screw coupler 128 (shown elsewhere - illustratively, the plurality of ledges 138). The first mixing chamber 142 and the shuttle 160 form a helical coupling for movably coupling to each other. Illustratively, the shuttle 160 includes a helically extending ridge 166 and the first mixing chamber 142 includes a helically extending groove 168 that receives the ridge 166. The shuttle 160 includes an actuation feature (illustratively, two radially-outwardly extending fingers 170) that, as described in further detail below, engage and are driven by the actuation feature of the proximal housing portion 30 (shown elsewhere - illustratively, the two helically extending ramps 38). Internally, the shuttle 160 includes a first restraining feature (illustratively, eight radially-inwardly extending tabs 172, four of which are shown in FIG. 23) that engages the mixing piston 158. Illustratively, the shuttle 160 also includes channels 174 disposed between adjacent tabs 172 (three of which are shown in FIG. 23). The mixing piston 158 includes a second restraining feature (illustratively, eight radially-outwardly extending tabs 176) that engages the first restraining feature of the shuttle 160. Initially and as shown in FIG. 19, the first restraining feature engages the second restraining feature (illustratively, the radially-inwardly extending tabs 172 of the shuttle 160 are angularly aligned with and engage the radially-outwardly extending tabs 176 of the mixing piston 158) to hold the mixing piston 158 in a position between the first mixing chamber 142 and the second mixing chamber 144. The mixing piston 158 thereby maintains separation of reagents in the first mixing chamber 142 and the second mixing chamber 144. Initially the actuator spring 156 is also compressed within the second mixing chamber 144 against the mixing piston 158. In a subsequent configuration, as described in further detail below, the shuttle 160 rotates relative to the first mixing chamber 142 and the second mixing chamber 144 to disengage the first restraining feature from the second restraining feature (illustratively, the radially-inwardly extending tabs 172 of the shuttle 160 are angularly misaligned with, or angularly offset from, the radially-outwardly extending tabs 176 of the mixing piston 158, and the channels 174 are angularly aligned with the radially-outwardly extending tabs 176 of the mixing piston 158). As a result, the actuator spring 156 expands and moves the mixing piston 158 into the shuttle 160 and the first mixing chamber 142, which permits the reagents in the first mixing chamber 142 and the second mixing chamber 144 to mix. Mixing of the reagents generates one or more pressurized fluids (for example, one or more gases), and the pressurized fluid(s) are delivered to other components of the therapeutic agent delivery assembly 16.

In some embodiments, pressure generating actuators 140 have different structures. For example, suitable pressure generating actuators 140 include those described in: U.S. Patent No. 9,795,740 titled "Chemical Engines and Methods for Their Use, Especially in the Injection of Highly Viscous Fluids"; U.S. Publication No.2020/0030537, titled "Processes and Devices for Delivery of Fluid by Chemical Reaction"; and International Publication No. WO2019/050791, titled "System for Controlling Gas Generation with a Drug Delivery Device", the disclosures of which are expressly incorporated herein by reference in their entireties.

Any suitable chemical reagent or reagents can be used to generate one or more pressurized fluids in pressure generating actuators 140 of the present disclosure. Examples of generated gases include carbon dioxide gas, nitrogen gas, oxygen gas, chlorine gas, etc. Desirably, the generated gas is inert and non-flammable. The amount of gas needed to facilitate movement of other components of the therapeutic agent delivery assembly 16 may impact the type, amount, and concentration of each reagent used in pressure generating actuators 140. The reagents may be in dry form (for example, powdered form, tablet form) and/or in liquid form.

In one exemplary embodiment, a bicarbonate (which may be present in dry form) reacts with an acid (which may be present in liquid form) to produce carbon dioxide gas in pressure generating actuators 140. Examples of suitable bicarbonates include sodium bicarbonate, potassium bicarbonate, and ammonium bicarbonate. Other ingredients may also be present along with the bicarbonates, such as diatomaceous earth. Examples of suitable acids include acetic acid, citric acid, potassium bitartrate, disodium pyrophosphate, and calcium dihydrogen phosphate. In one particular example, the bicarbonate is potassium bicarbonate and the acid is aqueous citric acid, which may react to produce carbon dioxide gas and a liquid mixture of water and dissolved potassium citrate.

In some embodiments, other reactions may be used. In one example, a metal carbonate, such as copper carbonate or calcium carbonate, is thermally decomposed to produce carbon dioxide gas and the corresponding metal oxide in pressure generating actuators 140. In another example, 2,2'-azobisisobutyronitrile (AIBN) is heated to produce nitrogen gas in pressure generating actuators 140. In yet another example, enzymes (for example yeast) are reacted with sugar to produce carbon dioxide gas in pressure generating actuators 140. Some substances readily sublime, going from solid to gas. Such substances include but are not limited to naphthalene and iodine. In still yet another example, hydrogen peroxide is decomposed with catalysts such as enzymes (for example catalase) or manganese dioxide to produce oxygen gas in pressure generating actuators 140. In still yet another example, silver chloride is decomposed through exposure to light to generate a gas in pressure generating actuators 140. Suitable reagents, chemical formulations, and reactions are further described in the above-incorporated U.S. Patent No. 9,795,740, U.S. Publication No.2020/0030537, and International Publication No. WO2019/050791.

As described briefly above, the outlet 152 of the pressure generating actuator 140 may carry one or more absorbent materials 154. Such absorbent materials 154 may absorb excess liquid provided by mixing the reagents within the pressure generating actuator 140. Suitable absorbent materials are further described in the above-incorporated U.S. Publication No.2020/0030537.

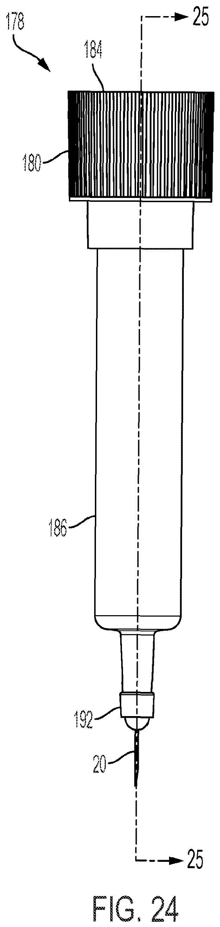

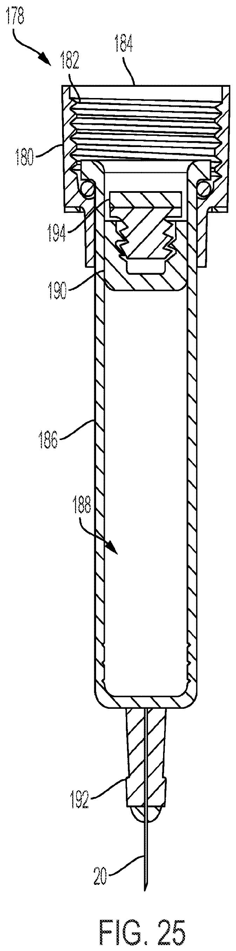

FIGS. 24 and 25 illustrate a syringe assembly 178 of the therapeutic agent delivery assembly 16. The syringe assembly 178 includes an inlet portion 180, and the inlet portion 180 includes an inlet coupling feature (illustratively, an internally threaded surface 182) that couples to the outlet coupling feature of the pressure generating actuator 140 (shown elsewhere - illustratively, the externally threaded surface 150). The inlet portion 180 also includes an inlet 184 that receives pressurized fluid(s) from the outlet 152 of the pressure generating actuator 140. The inlet portion 180 couples to a syringe chamber 186, and the syringe chamber 186 includes a syringe passageway 188 that receives the pressurized fluid(s) from the inlet portion 180. The syringe passageway 188 carries a syringe piston 190, and the syringe piston 190 translates away from the inlet portion 180 and towards an outlet portion 192 of the syringe assembly 178 when the syringe passageway 188 receives the pressurized fluid(s). Illustratively and as described in further detail below, the syringe piston 190 carries a magnetic component 194, which may also be referred to as a target, that facilitates determining the position of the syringe piston 190 in the syringe passageway 188. The syringe passageway 188 also carries the therapeutic agent 18 (shown elsewhere - illustratively, 2.25mL of the therapeutic agent, although other volumes, including, for example, 0.5mL, 1.0mL, 3.0mL or 5.0mL may alternatively be carried) between the syringe piston 190 and the outlet portion 192, more specifically the needle 20. As such, translation of the syringe piston 190 in the syringe passageway 188 causes the needle 20 to discharge the therapeutic agent therefrom. In other embodiments, different arrangements are possible. For example, the inlet portion 180 and the syringe chamber 186 may be monolithically formed with each other, or the syringe assembly 178 could be replaced by another type of therapeutic agent container, such as a bellows or bladder structure.

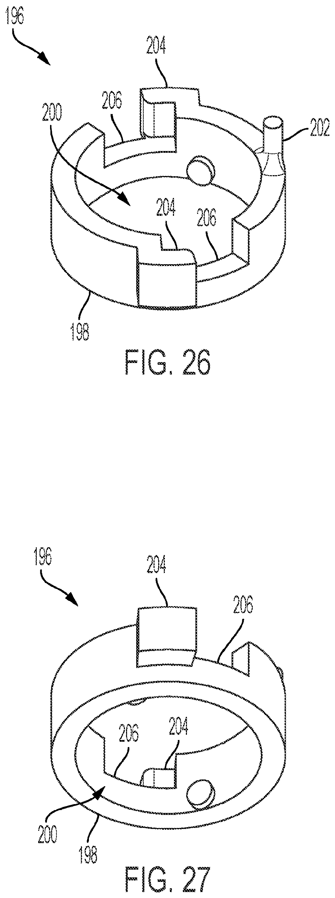

FIGS. 26 and 27 illustrate a restraint device 196 of the drive mechanism 28. The restraint device 196 includes a main body 198 that has a generally annular shape. The main body 198 includes an inner passageway 200 that carries other components of the drive mechanism 28. The main body 198 also includes a coupling feature (illustratively, a proximally extending post 202) for receiving the loop 78 of the wire 68 (shown elsewhere). The main body 198 further includes a restraint feature for selectively inhibiting motion of another component of the drive mechanism 28, as described further below. Illustratively, the restraint feature includes two pairs of radially-outwardly disposed ledges 204 and adjacent radially extending openings 206. In other embodiments, different arrangements of the restraint device 196 are possible.

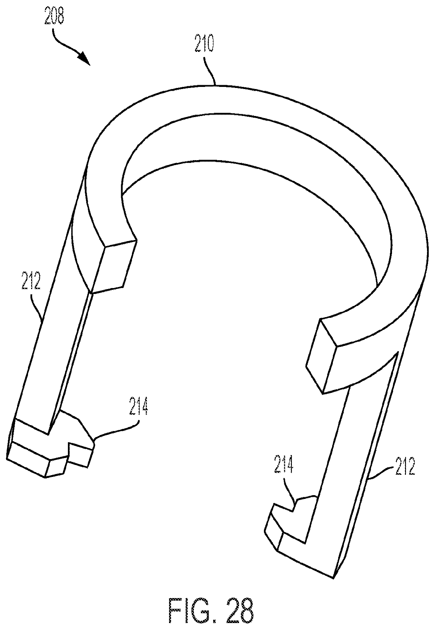

FIG. 28 illustrates a second latch device 208 of the drive mechanism 28. The second latch device 208 includes a coupling feature (illustratively, a semi-annular clip 210) for coupling the second latch device 208 to another component of the drive mechanism 28, as described further below. The second latch device 208 also includes a latch feature. Illustratively, the latch feature is provided by two opposed, distally extending legs 212 that terminate with feet 214. The second latch device 208 may be prestressed such that the legs 212 and feet 214 are urged radially outwardly from the configuration shown in FIG. 28. As described in further detail below, the legs 212 and feet 214 thereby disengage another component of the drive mechanism 28 when the legs 212 are unconstrained in a radial direction (illustratively, when the legs 212 and feet 214 disengage the ledges 204 and align with the openings 206 of the restraint device 196 - all shown elsewhere). In other embodiments, different arrangements of the second latch device 208 are possible.

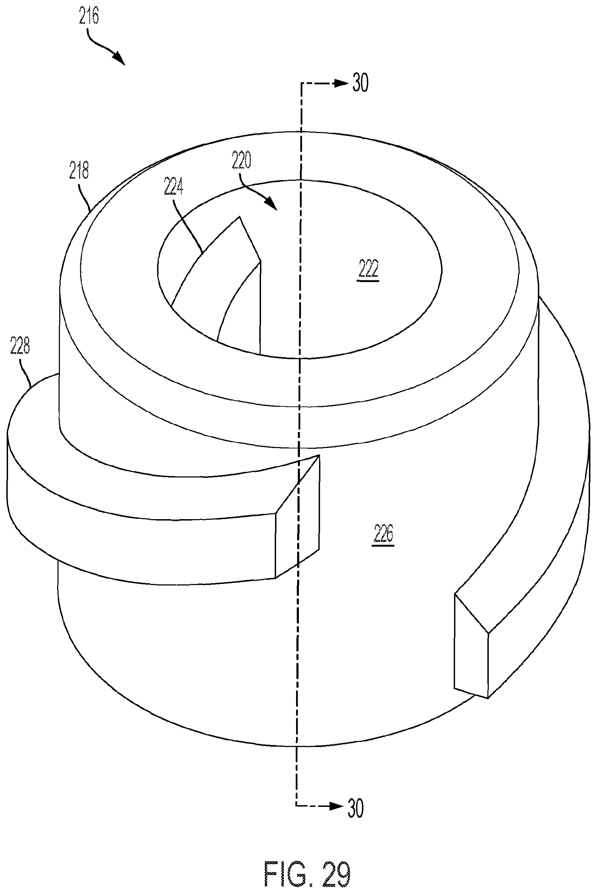

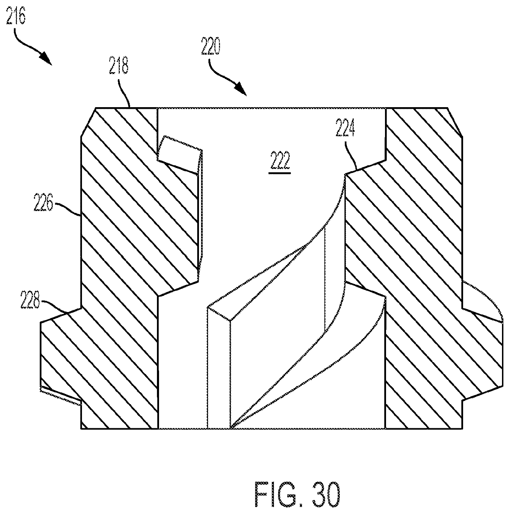

FIGS. 29 and 30 illustrate a second catch device 216 of the drive mechanism 28. The second catch device 216 is illustratively shown as being a threaded nut. That is, the second catch device 216 illustratively includes a main body 218 that has a generally annular shape. The main body 218 includes an inner passageway 220, and an inner surface 222 of the inner passageway 220 includes a first internal threaded surface 224. The first internal threaded surface 224 threadably engages the first external threaded surface 114 of the drive screw 110 (shown elsewhere). As such, the first internal threaded surface 224 also has the first thread direction, illustratively the left-handed thread direction. An outer surface 226 of the second catch device 216 includes a second external threaded surface 228. The second external threaded surface 228 threadably engages another component of the drive mechanism 28, as described further below. The second external threaded surface 228 has a second thread direction that is opposite the first thread direction. Illustratively, the second thread direction is a right-handed thread direction. The second catch device 216 includes a catch feature for detachably engaging the latch feature of the second latch device 208 (shown elsewhere). Illustratively, the second external threaded surface 228 provides the catch feature, and the second external threaded surface 228 engages the legs 212 of the second latch device 208. In other embodiments, different arrangements of the second catch device 216 are possible.

As described in further detail below, the second catch device 216, the second latch device 208, the restraint device 196, and the wire 68 together form a second release device of the drive mechanism 28. More specifically, the wire 68 and the restraint device 196 facilitate detachment or release of the latch feature of the second latch device 208 (illustratively, the legs 212) from the catch feature of the second catch device 216 (illustratively, the second external threaded surface 228) to facilitate actuation of the drive mechanism 28, more specifically to facilitate reconfiguration of the system 10 from the deployed configuration to the retracted configuration.

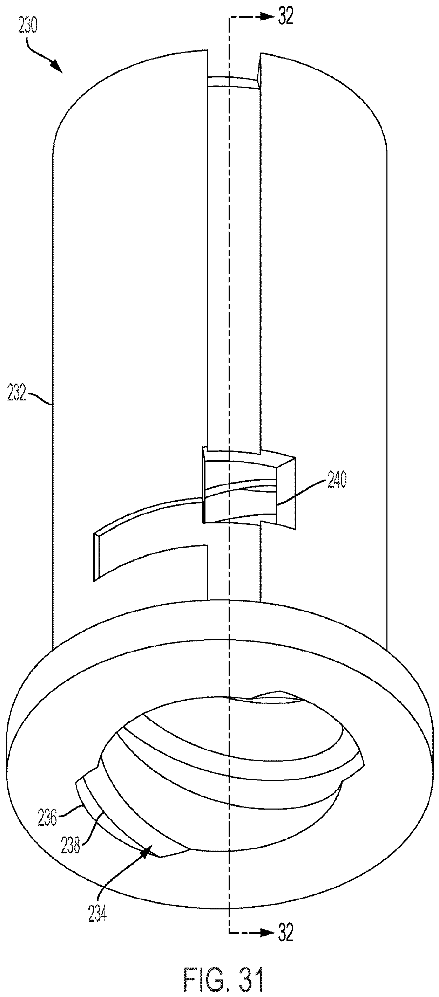

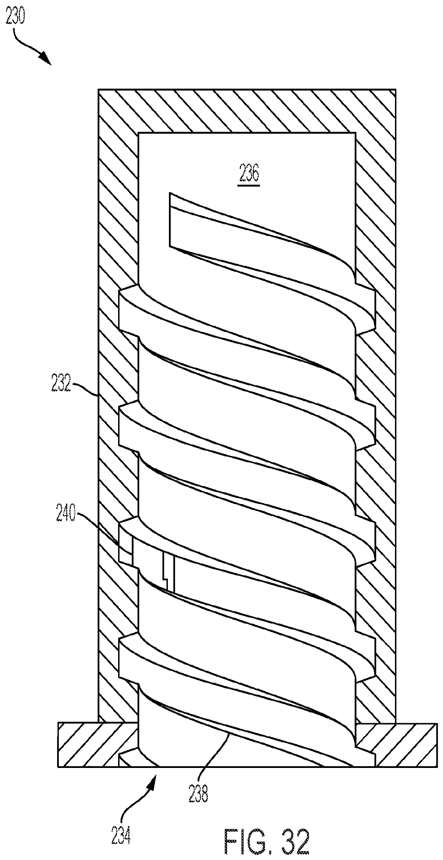

FIGS. 31 and 32 illustrate a retraction cap 230 of the drive mechanism 28. The retraction cap 230 includes a main body 232 that has a generally closed-cylindrical shape. The main body 232 includes an inner chamber 234, and an inner surface 236 of the inner chamber 234 includes a second internal threaded surface 238. The second internal threaded surface 238 threadably engages the second external threaded surface 228 of the second catch device 216 (shown elsewhere). As such, the second internal threaded surface 238 also has the second thread direction, illustratively the right-handed thread direction. The main body 232 includes further includes two radially-extending openings 240, one of which is shown in FIGS. 31 and 32. As described in further detail below, the feet 214 of the second latch device 208 (shown elsewhere) initially extend through the openings 240 to engage the second catch device 216.

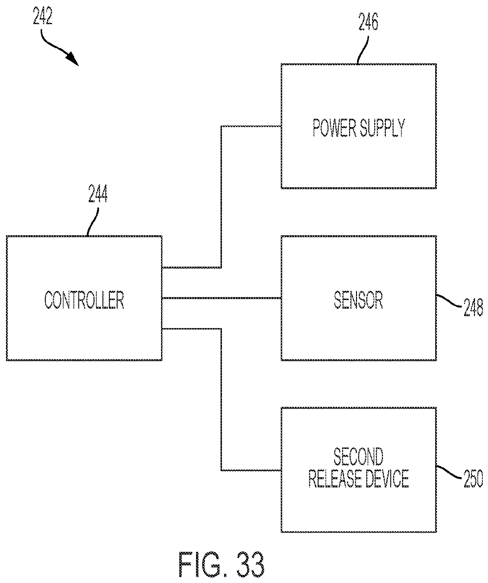

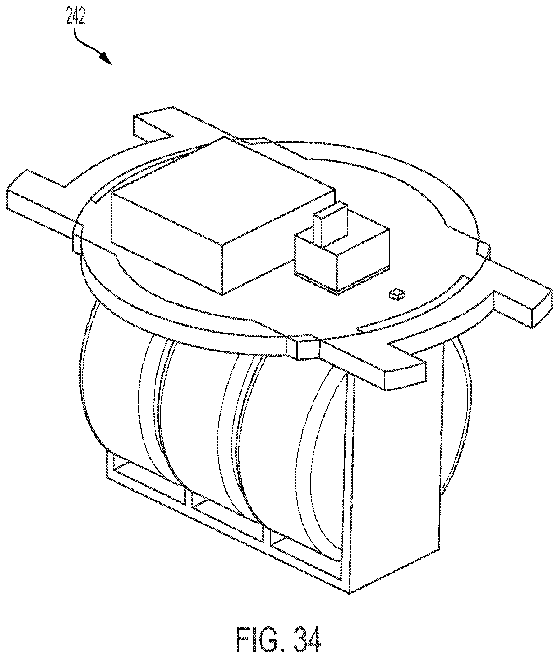

FIGS. 33 and 34 illustrate an electronics assembly 242 of the therapeutic agent delivery system 10. The electronics assembly 242 includes an electronic controller 244 that is operatively coupled to and receives power from a power supply 246 (illustratively, a battery). The controller 244 is also operatively coupled to a sensor 248. The sensor 248 may send a sensor signal to the controller 244 in response to sensing various types of inputs. For example, the sensor 248 may be configured to determine the position of the syringe piston 190 in the syringe chamber 186 (both shown elsewhere - for example, to determine if the syringe piston 190 has been moved toward the syringe outlet portion 192, thereby indicating that the therapeutic agent has been discharged from the needle 20). More specifically, the sensor 248 may be a hall effect sensor that is configured to sense the magnetic component 194 (shown elsewhere) carried by the syringe piston 190. As another example, the sensor 248 and/or target 194 may be an optical sensor or a vibration sensor. As yet another example, the sensor 248 may be configured to sense and send the sensor signal to the controller 244 upon actuation of the user input 26 (shown elsewhere).

With specific reference to FIGS. 33, the controller 244 is also operatively coupled to the second release device 250 of the drive mechanism 28. The controller 244 may send a retraction signal to the second release device 250 (for example, upon receiving the sensor signal, or after a predetermined time period if the sensor 248 senses when the user input 26 is actuated) to actuate the second release device 250. Illustratively, the retraction signal may be an electric current, and the wire 68 of the second release device 250 (shown elsewhere) may comprise one or more shape memory materials that contract upon receiving thermal energy, such as nitinol. As such, the wire 68 is heated and contracts upon receiving the electric current from the controller 244, which actuates the second release device 250. The drive mechanism 28 thereby reconfigures the system 10 from the deployed configuration to the retracted configuration. Further details of these actions are described below. Alternatively, the electronics assembly 242 could take other forms. For example, the controller 244 could provide the retraction signal to a separate heating element (not shown), and the heating element could provide thermal energy to the wire 68 to thereby contract the wire 68.

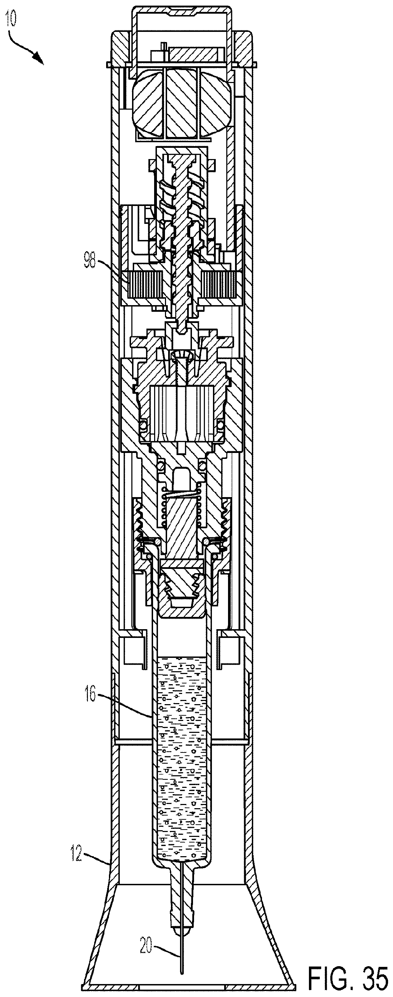

Illustratively, actuation of the therapeutic agent delivery system 10 is as follows. Referring to FIG. 35, the therapeutic agent delivery system 10 is illustrated in an initial or first configuration. In the first configuration, the therapeutic agent delivery assembly 16 is disposed in the stowed configuration (illustratively, a configuration in which the needle 20 is disposed entirely within the housing 12). In the first configuration, the torsion spring 98 is held in a higher energy storage configuration (that is, a loaded configuration).

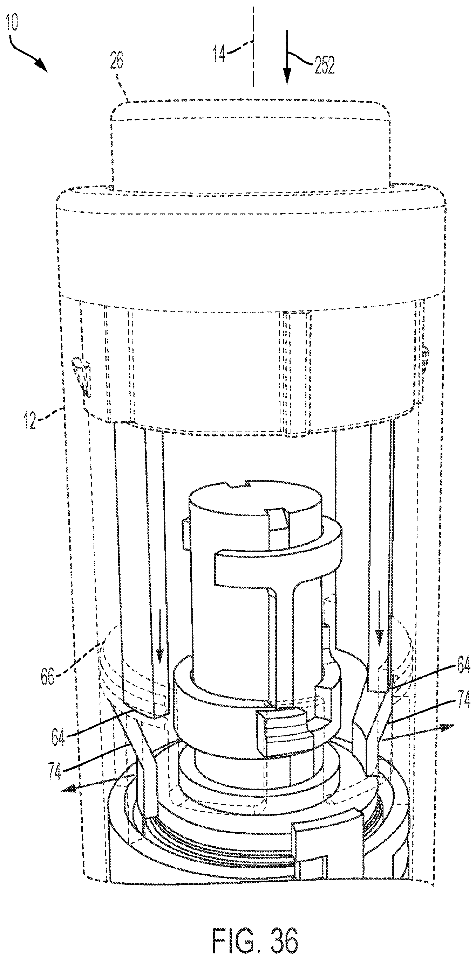

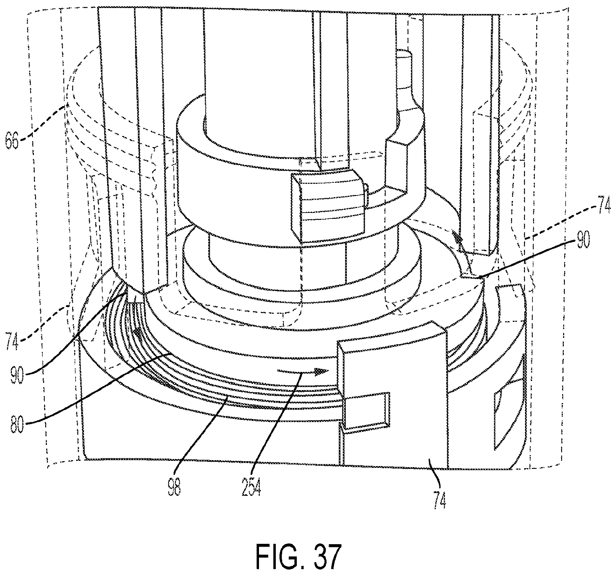

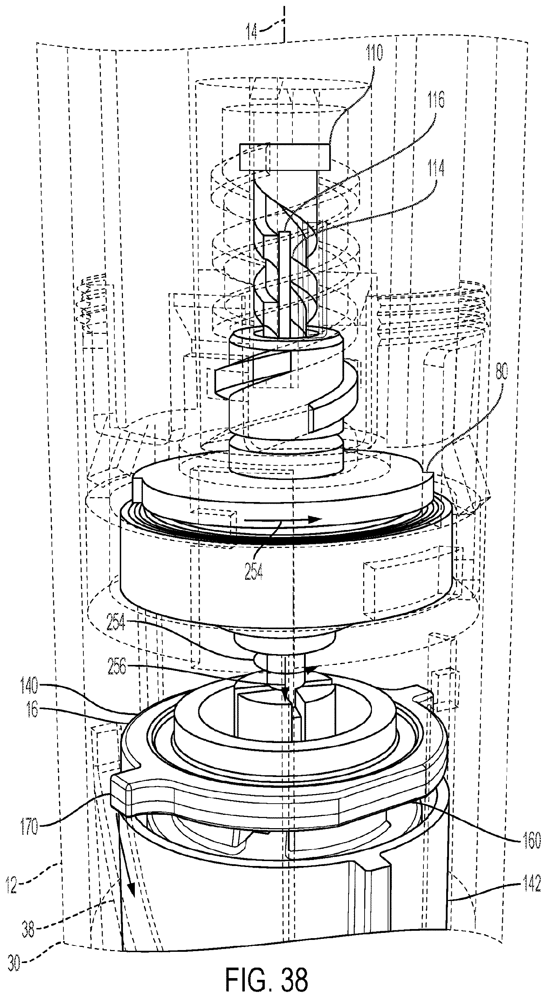

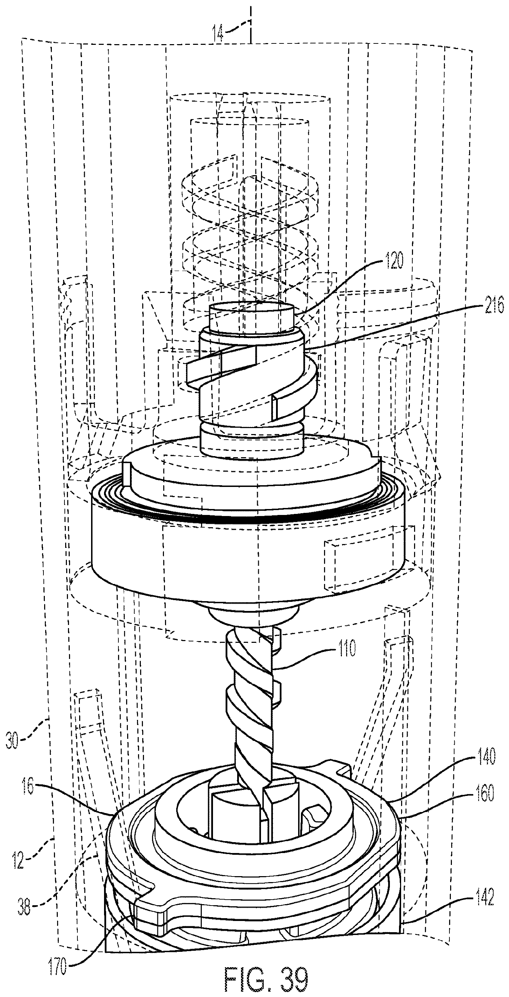

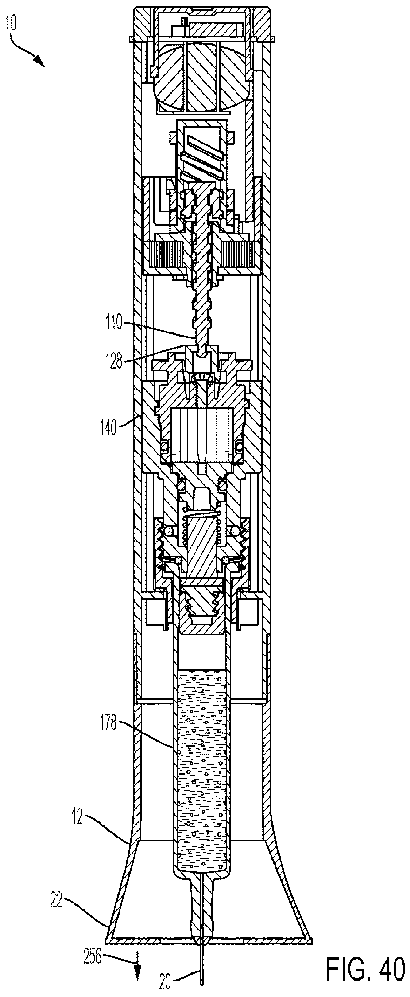

Referring to FIG. 36, the user input 26 and the legs 74 of the first latch device 66 are illustrated in the first configuration. In this configuration, the user input 26 may be actuated by a user to actuate the therapeutic agent delivery system 10. More specifically, the user input 26 may be pressed and translated in a direction 252 (which may be, for example, substantially parallel to the longitudinal axis 14 (that is, parallel ± 5 degrees)) relative to the housing 12. This action causes the actuation surfaces 64 of the user input 26 to engage the legs 74 of the first latch device 66. This action in turn causes, as shown in FIG. 37, the legs 74 of the first latch device 66 to deflect radially outwardly and disengage the transversely facing surfaces 90 of the first catch device 80. As a result, the torsion spring 98 is unconstrained and unwinds to release stored energy. That is, the torsion spring 98 reconfigures from the higher energy storage configuration to an intermediate energy storage configuration. The torsion spring 98 thereby rotates the first catch device 80 in a direction 254. As shown in FIG. 38, the drive screw 110 also rotates together with the first catch device 80 in the direction 254 due to engagement of the shoulder surfaces 88 of the first catch device 80 (shown elsewhere) and the cutout 116 of the drive screw 110. The drive screw 110 also distally translates relative to the first catch device 80 in direction 256 (which may be, for example, substantially parallel to the longitudinal axis 14 (that is, parallel ± 5 degrees)) due to threaded engagement between the first external threaded surface 114 of the drive screw 110 and the first internal threaded surface 224 of the second catch device 216 (shown elsewhere). The drive screw 110 continues translating in the direction 256 until, as shown in FIG. 39, the enlarged head 120 of the drive screw 110 engages the second catch device 216. As shown in FIG. 40, translation of the drive screw 110 in the direction 256 causes translation of the drive screw coupler 128, the pressure generating actuator 140, and the syringe assembly 178 in the direction 256. As a result, the system 10 moves to the deployed configuration and the needle 20 is exposed at a distal end portion 22 of the housing 12.

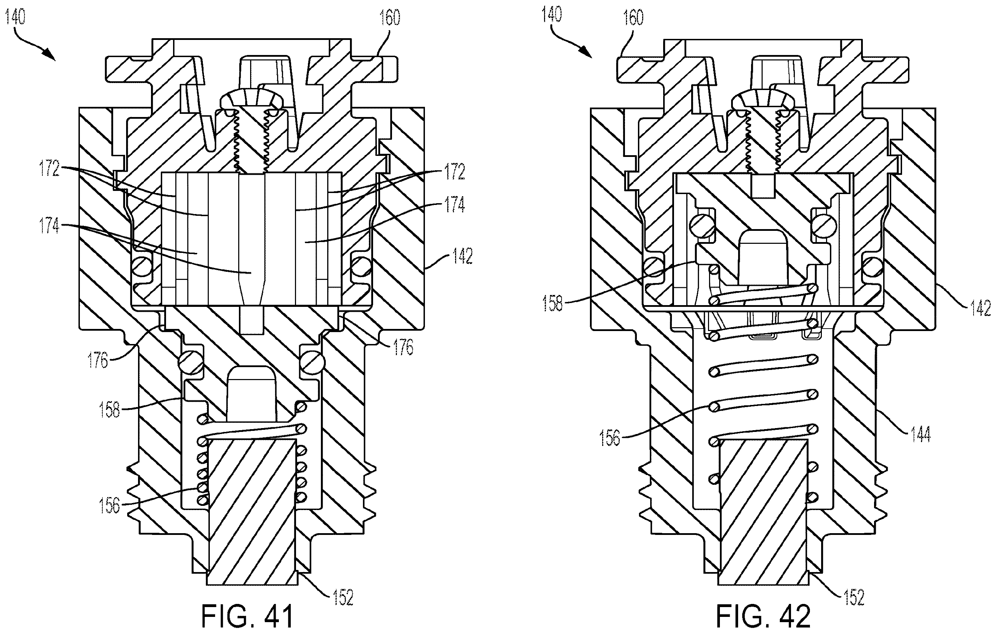

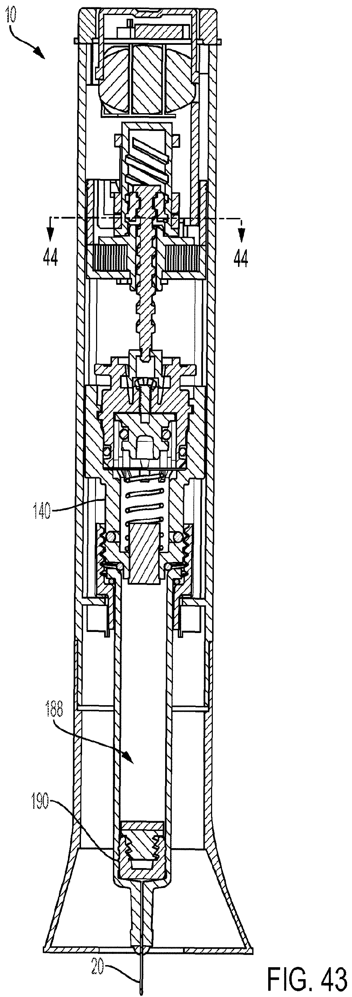

Referring again to FIGS. 38 and 39, translation of the therapeutic agent delivery assembly 16 distally relative to the housing 12 also causes the radially-outwardly extending fingers 170 of the shuttle 160 to engage and slide over the helically extending ramps 38 of the proximal housing portion 30. This engagement causes the shuttle 160 to rotate relative to the mixing chambers 142, 144 of the pressure generating actuator 140 (illustratively, about an axis that is substantially parallel to the longitudinal axis 14 (that is, parallel ± 5 degrees)), which actuates the pressure generating actuator 140. More specifically and as illustrated in FIG. 41, rotating the shuttle 160 relative to the first and second mixing chambers 142, 144 angularly misaligns the radially-inwardly extending tabs 172 of the shuttle 160 with the radially-outwardly extending tabs 176 of the mixing piston 158 and angularly aligns the channels 174 of the shuttle 160 with the radially-outwardly extending tabs 176 of the mixing piston 158. As a result, the actuator spring 156 is relatively unconstrained and, as shown in FIG. 42, the actuator spring 156 expands and translates the mixing piston 158 into the shuttle 160 and the first mixing chamber 142. The reagents in the first mixing chamber 142 and the second mixing chamber 144 then mix and react to provide a pressurized gas, which the pressure generating actuator 140 delivers from the actuator outlet 152. Referring to FIG. 43, the pressure generating actuator 140 delivers the pressurized gas to the syringe passageway 188, which translates the syringe piston 190 distally within the syringe passageway 188. As such, the syringe piston 190 pushes the therapeutic fluid distally to the needle 20, and the needle 20 discharges the therapeutic fluid and delivers the therapeutic fluid to the subject.

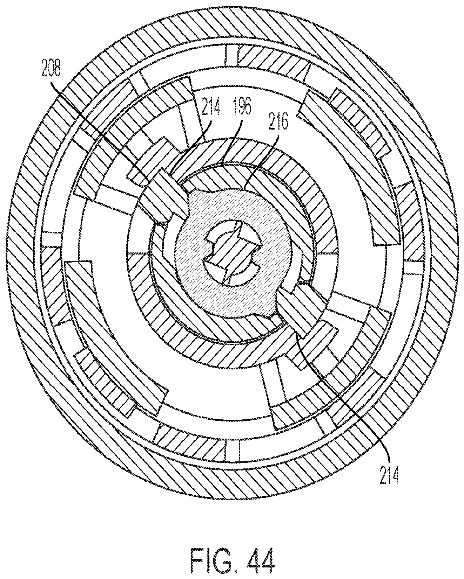

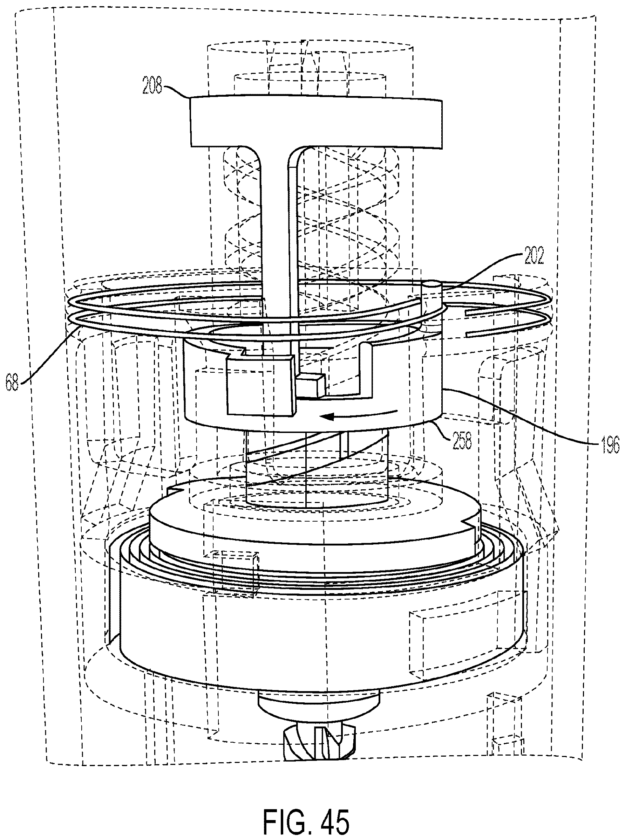

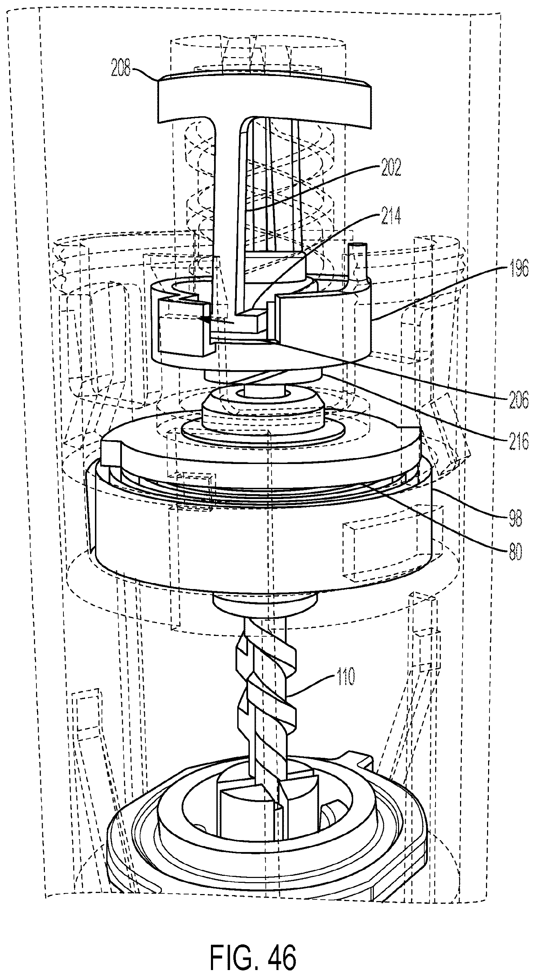

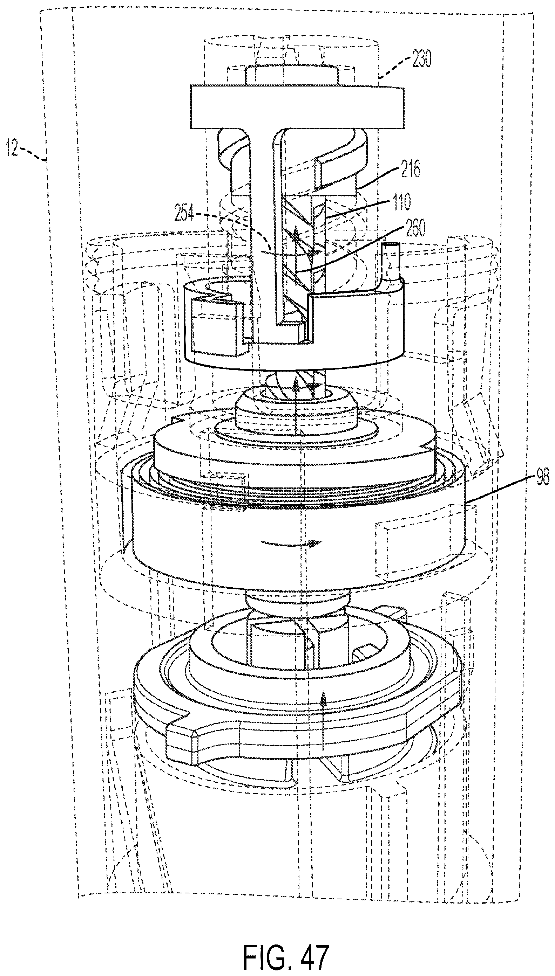

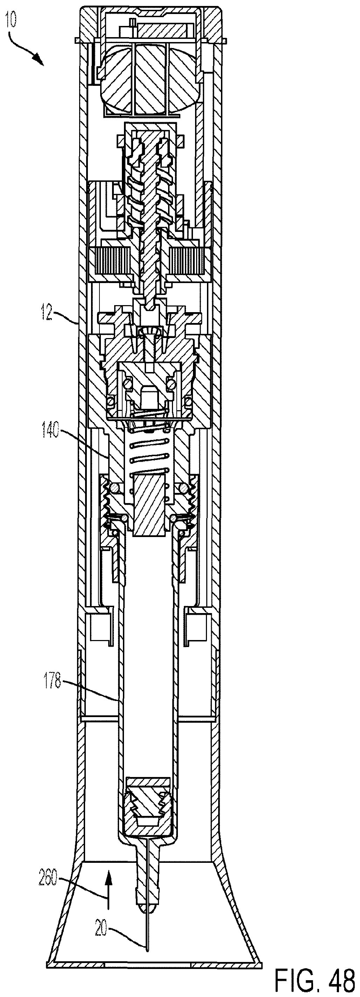

During the above actions, the second release device 250 remains in its initial configuration. More specifically and as shown in FIG. 44, the feet 214 of the second latch device 208 are held by the restraint device 196 in engagement with the second catch device 216. After delivering the therapeutic fluid to the subject (illustratively, determined by the sensor 248 sensing that the magnetic component 194, and the syringe piston 190, are disposed near the outlet portion 192 of the syringe assembly 178 - all shown elsewhere), the electronics assembly 242 actuates the second release device 250 (both shown elsewhere). More specifically and referring to FIG. 45, the electronics assembly 242 (shown elsewhere) delivers an electric current to the wire 68 to heat and thereby contract the wire 68. The wire 68 thereby pulls the post 202 of the restraint device 196, and the restraint device 196 rotates in direction 258 relative to the second latch device 208. Referring to FIG. 46, when the openings 206 of the restraint device 196 align with the legs 212 and the feet 214 of the second latch device 208, the legs 212 deflect outwardly and the feet 214 disengage the second catch device 216. As a result, the second catch device 216 and the torsion spring 98, due to its connection to the second catch device 216 via the first catch device 80 and the drive screw 110, are rotatably unconstrained. The torsion spring 98 thereby unwinds to release stored energy. That is, the torsion spring 98 reconfigures from the intermediate energy storage configuration to a lower energy storage configuration. As shown in FIG. 47, the torsion spring 98 rotates the drive screw 110 and the second catch device 216 in direction 254, which proximally translates the drive screw 110 and the second catch device 216 in direction 260 relative to the retraction cap 230 and the housing 12 (due to the second catch device 216 and retraction cap 230 being coupled by "opposite handed" threads than the drive screw 110 and the second catch device 216). As shown in FIG. 48, this action causes the pressure generating actuator 140 and the syringe assembly 178 to proximally translate in direction 260 relative to the housing 12. That is, the system 10 moves to the retracted configuration and the needle 20 is disposed entirely within the housing 12. Illustratively, the therapeutic agent delivery system 10 cannot be actuated again (that is, the therapeutic agent delivery system 10 may be "locked out), and the therapeutic agent delivery system 10 may be discarded.

While this invention has been described as having exemplary designs, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

The claims of the parent application are reproduced immediately below as clauses. These clauses define preferred embodiments. The applicant reserves the right to pursue protection for subject-matter contained in the parent application as filed, either in the present divisional application or in a further application divided from the present divisional application.
1. A therapeutic agent delivery system, comprising:
   a housing having a distal end portion;
   a therapeutic agent delivery assembly carried by the housing, the therapeutic agent delivery assembly comprising:
      a chamber comprising a first passageway configured to carry a therapeutic agent;
      a needle in communication with the first passageway;
   the therapeutic agent delivery assembly being translatable relative to the housing from a stowed configuration to a deployed configuration, in the deployed configuration the needle at least partially extending distally from the distal end portion of the housing, and the therapeutic agent delivery assembly being translatable relative to the housing from the deployed configuration to a retracted configuration, in the retracted configuration the needle being disposed proximally relative to the distal end portion of the housing;
   a drive mechanism carried by the housing, the drive mechanism comprising:
      a torsion spring;
      a first release device being actuatable to permit the torsion spring to release energy and reconfigure from a higher energy storage configuration to an intermediate energy storage configuration, the drive mechanism thereby translating the therapeutic agent delivery assembly from the stowed configuration to the deployed configuration; and
      a second release device being actuatable to permit the torsion spring to further release energy and reconfigure from the intermediate energy storage configuration to a lower energy storage configuration, the drive mechanism thereby translating the therapeutic agent delivery assembly from the deployed configuration to the retracted configuration.
2. The therapeutic agent delivery system of clause 1, further comprising a user input carried by the housing and configured to be actuated by a user, upon actuation the user input thereby actuating the first release device.
3. The therapeutic agent delivery system of clause 2, further comprising an electronics assembly carried by the housing and operably coupled to the second release device, the electronics assembly sending a retraction signal to actuate the second release device.
4. The therapeutic agent delivery system of clause 3, wherein the second release device comprises a wire, and the second release device is actuatable by contracting the wire.
5. The therapeutic agent delivery system of any of clauses 1-4, further comprising the therapeutic agent carried in the first passageway of the chamber.
6. A therapeutic agent delivery system, comprising:
   a housing having a distal end portion;
   a therapeutic agent delivery assembly carried by the housing, the therapeutic agent delivery assembly comprising:
      a chamber comprising a first passageway configured to carry a therapeutic agent;
      a needle in communication with the first passageway;
   the therapeutic agent delivery assembly being translatable relative to the housing from a stowed configuration to a deployed configuration, in the deployed configuration the needle at least partially extending distally from the distal end portion of the housing, and the therapeutic agent delivery assembly being translatable relative to the housing from the deployed configuration to a retracted configuration, in the retracted configuration the needle being disposed proximally relative to the distal end portion of the housing;
   a drive mechanism carried by the housing, the drive mechanism comprising:
      a threaded nut;
      a drive screw carried by and threadably coupled to the threaded nut, the drive screw being rotatable and translatable relative to the housing to translate the therapeutic agent delivery assembly relative to the housing; and
      a torsion spring, the torsion spring releasing energy to rotate and translate the drive screw relative to the housing, the drive mechanism thereby translating the therapeutic agent delivery assembly from the stowed configuration to the deployed configuration and from the deployed configuration to the retracted configuration.
7. The therapeutic agent delivery system of clause 6, wherein the drive mechanism further comprises:
   a first release device being actuatable to permit the torsion spring to release energy and reconfigure from a higher energy storage configuration to an intermediate energy storage configuration, the drive mechanism thereby translating the therapeutic agent delivery assembly from the stowed configuration to the deployed configuration; and
   a second release device being actuatable to permit the torsion spring to further release energy and reconfigure from the intermediate energy storage configuration to a lower energy storage configuration, the drive mechanism thereby translating the therapeutic agent delivery assembly from the deployed configuration to the retracted configuration.
8. The therapeutic agent delivery system of clause 7, wherein the electronics assembly comprises a sensor configured to sense discharge of the therapeutic agent from the needle, and the electronics assembly being configured to send the retraction signal upon the sensor sensing discharge of the therapeutic agent from the needle.
9. The therapeutic agent delivery system of any of clauses 6-8, wherein the drive mechanism further comprises a retraction cap carrying the threaded nut, wherein the drive screw comprises a first external threaded surface, the threaded nut comprises a first internal threaded surface that threadably engages the first external threaded surface, both of the first external threaded surface and the first internal threaded surface having a first thread direction, and wherein the threaded nut further comprises a second external threaded surface, the retraction cap comprises a second internal threaded surface that threadably engages the second external threaded surface, both of the second external threaded surface and the second internal threaded surface having a second thread direction opposite the first thread direction.
10. The therapeutic agent delivery system of any of clauses 6-9, wherein the torsion spring is a spiral-wound torsion spring.
11. A therapeutic agent delivery system, comprising:
   a housing having a distal end portion;
   a therapeutic agent delivery assembly carried by the housing, the therapeutic agent delivery assembly comprising:
      a chamber comprising a first passageway;
      a therapeutic agent carried in the first passageway;
      a needle in communication with the first passageway;
   the therapeutic agent delivery assembly being translatable relative to the housing from a stowed configuration to a deployed configuration, in the deployed configuration the needle at least partially extending distally from the distal end portion of the housing, and the therapeutic agent delivery assembly being translatable relative to the housing from the deployed configuration to a retracted configuration, in the retracted configuration the needle being disposed proximally relative to the distal end portion of the housing;
   a drive mechanism carried by the housing, the drive mechanism comprising:
      a retraction cap;
      a threaded nut carried by and threadably coupled to the retraction cap;
      a drive screw carried by and threadably coupled to the threaded nut, the drive screw being rotatable and translatable relative to the housing to translate the therapeutic agent delivery assembly relative to the housing; and
      a rotary actuator configured to drive the drive screw in a first rotary direction;
      wherein the drive screw comprises a first external threaded surface, the threaded nut comprises a first internal threaded surface that threadably engages the first external threaded surface, both of the first external threaded surface and the first internal threaded surface having a first thread direction, wherein the threaded nut further comprises a second external threaded surface, the retraction cap comprises a second internal threaded surface that threadably engages the second external threaded surface, both of the second external threaded surface and the second internal threaded surface having a second thread direction opposite the first thread direction, wherein the rotary actuator is configured to drive the drive screw in the first rotary direction and first thereby translate the therapeutic agent delivery assembly from the stowed configuration to the deployed configuration due to the first thread direction, and second thereby translate the therapeutic agent delivery assembly from the deployed configuration to the retracted configuration due to the second thread direction.
12. The therapeutic agent delivery system of clause 11, wherein the rotary actuator is a torsion spring.
13. The therapeutic agent delivery system of clause 12, wherein the torsion spring is a spiral-wound torsion spring.
14. The therapeutic agent delivery system of any of clauses 12 and 13, wherein the drive mechanism further comprises:
   a first release device being actuatable to permit the torsion spring to release energy and reconfigure from a higher energy storage configuration to an intermediate energy storage configuration, the torsion spring thereby driving the drive screw in the first rotary direction and translating the therapeutic agent delivery assembly from the stowed configuration to the deployed configuration due to the first thread direction; and
   a second release device being actuatable to permit the torsion spring to further release energy and reconfigure from the intermediate energy storage configuration to a lower energy storage configuration, the torsion spring thereby driving the drive screw in the first rotary direction and translating the therapeutic agent delivery assembly from the deployed configuration to the retracted configuration due to the second thread direction.
15. The therapeutic agent delivery system of clause 14, further comprising a user input carried by the housing and configured to be actuated by a user, upon actuation the user input thereby actuating the first release device.
16. The therapeutic agent delivery system of clause 15, further comprising an electronics assembly carried by the housing and operably coupled to the second release device, the electronics assembly sending a retraction signal to actuate the second release device.
17. The therapeutic agent delivery system of any of clauses 15 and 16, wherein the second release device comprises a wire, and the second release device is actuatable by contracting the wire.
18. The therapeutic agent delivery system of clause 17, wherein the wire comprises one or more shape memory materials and contracts upon receiving thermal energy.

## Claims

1. A therapeutic agent delivery system, comprising:
a housing having a distal end portion;
a therapeutic agent delivery assembly carried by the housing, the therapeutic agent delivery assembly comprising:
a chamber comprising a first passageway configured to carry a therapeutic agent;
a needle in communication with the first passageway;
the therapeutic agent delivery assembly being translatable relative to the housing from a stowed configuration to a deployed configuration, in the deployed configuration the needle at least partially extending distally from the distal end portion of the housing, and the therapeutic agent delivery assembly being translatable relative to the housing from the deployed configuration to a retracted configuration, in the retracted configuration the needle being disposed proximally relative to the distal end portion of the housing;
a drive mechanism carried by the housing, the drive mechanism comprising:
a threaded nut;
a drive screw carried by and threadably coupled to the threaded nut, the drive screw being rotatable and translatable relative to the housing to translate the therapeutic agent delivery assembly relative to the housing; and
a torsion spring, the torsion spring releasing energy to rotate and translate the drive screw relative to the housing, the drive mechanism thereby translating the therapeutic agent delivery assembly from the stowed configuration to the deployed configuration and from the deployed configuration to the retracted configuration.

2. The therapeutic agent delivery system of claim 1, wherein the drive mechanism further comprises:
a first release device being actuatable to permit the torsion spring to release energy and reconfigure from a higher energy storage configuration to an intermediate energy storage configuration, the drive mechanism thereby translating the therapeutic agent delivery assembly from the stowed configuration to the deployed configuration; and
a second release device being actuatable to permit the torsion spring to further release energy and reconfigure from the intermediate energy storage configuration to a lower energy storage configuration, the drive mechanism thereby translating the therapeutic agent delivery assembly from the deployed configuration to the retracted configuration.

3. The therapeutic agent delivery system of claim 2, wherein the electronics assembly comprises a sensor configured to sense discharge of the therapeutic agent from the needle, and the electronics assembly being configured to send the retraction signal upon the sensor sensing discharge of the therapeutic agent from the needle.

4. The therapeutic agent delivery system of any of claims 1-3, wherein the drive mechanism further comprises a retraction cap carrying the threaded nut, wherein the drive screw comprises a first external threaded surface, the threaded nut comprises a first internal threaded surface that threadably engages the first external threaded surface, both of the first external threaded surface and the first internal threaded surface having a first thread direction, and wherein the threaded nut further comprises a second external threaded surface, the retraction cap comprises a second internal threaded surface that threadably engages the second external threaded surface, both of the second external threaded surface and the second internal threaded surface having a second thread direction opposite the first thread direction.

5. The therapeutic agent delivery system of any of claims 1-4, wherein the torsion spring is a spiral-wound torsion spring.

6. A therapeutic agent delivery system, comprising:
a housing having a distal end portion;
a therapeutic agent delivery assembly carried by the housing, the therapeutic agent delivery assembly comprising:
a chamber comprising a first passageway;
a therapeutic agent carried in the first passageway;
a needle in communication with the first passageway;
the therapeutic agent delivery assembly being translatable relative to the housing from a stowed configuration to a deployed configuration, in the deployed configuration the needle at least partially extending distally from the distal end portion of the housing, and the therapeutic agent delivery assembly being translatable relative to the housing from the deployed configuration to a retracted configuration, in the retracted configuration the needle being disposed proximally relative to the distal end portion of the housing;
a drive mechanism carried by the housing, the drive mechanism comprising:
a retraction cap;
a threaded nut carried by and threadably coupled to the retraction cap;
a drive screw carried by and threadably coupled to the threaded nut, the drive screw being rotatable and translatable relative to the housing to translate the therapeutic agent delivery assembly relative to the housing; and
a rotary actuator configured to drive the drive screw in a first rotary direction;
wherein the drive screw comprises a first external threaded surface, the threaded nut comprises a first internal threaded surface that threadably engages the first external threaded surface, both of the first external threaded surface and the first internal threaded surface having a first thread direction, wherein the threaded nut further comprises a second external threaded surface, the retraction cap comprises a second internal threaded surface that threadably engages the second external threaded surface, both of the second external threaded surface and the second internal threaded surface having a second thread direction opposite the first thread direction, wherein the rotary actuator is configured to drive the drive screw in the first rotary direction and first thereby translate the therapeutic agent delivery assembly from the stowed configuration to the deployed configuration due to the first thread direction, and second thereby translate the therapeutic agent delivery assembly from the deployed configuration to the retracted configuration due to the second thread direction.

7. The therapeutic agent delivery system of claim 6, wherein the rotary actuator is a torsion spring.

8. The therapeutic agent delivery system of claim 7, wherein the torsion spring is a spiral-wound torsion spring.

9. The therapeutic agent delivery system of any of claims 7 and 8, wherein the drive mechanism further comprises:
a first release device being actuatable to permit the torsion spring to release energy and reconfigure from a higher energy storage configuration to an intermediate energy storage configuration, the torsion spring thereby driving the drive screw in the first rotary direction and translating the therapeutic agent delivery assembly from the stowed configuration to the deployed configuration due to the first thread direction; and
a second release device being actuatable to permit the torsion spring to further release energy and reconfigure from the intermediate energy storage configuration to a lower energy storage configuration, the torsion spring thereby driving the drive screw in the first rotary direction and translating the therapeutic agent delivery assembly from the deployed configuration to the retracted configuration due to the second thread direction.

10. The therapeutic agent delivery system of claim 9, further comprising a user input carried by the housing and configured to be actuated by a user, upon actuation the user input thereby actuating the first release device.

11. The therapeutic agent delivery system of claim 10, further comprising an electronics assembly carried by the housing and operably coupled to the second release device, the electronics assembly sending a retraction signal to actuate the second release device.

12. The therapeutic agent delivery system of any of claims 10 and 11, wherein the second release device comprises a wire, and the second release device is actuatable by contracting the wire.

13. The therapeutic agent delivery system of claim 12, wherein the wire comprises one or more shape memory materials and contracts upon receiving thermal energy.
